# EUROPEAN PATENT SPECIFICATION

(11) **EP 3 610 262 B1**
(45) Date of publication and mention of the grant of the patent: **04.03.2026**
(21) Application number: 18715752.4
(22) Date of filing: 12.04.2018
(51) Int. Cl.: G01N 33/553, B01J 20/28, B03C 1/00, B01J 20/26, G01N 33/543

(54) **SUPERPARAMAGNETIC AND HIGHLY POROUS POLYMER PARTICLES FOR DIAGNOSTIC APPLICATIONS**
SUPERPARAMAGNETISCHE UND HOCHPORÖSE POLYMER PARTIKEL FÜR DIAGNOSTISCHE ANWENDUNGEN
PARTICULES POLYMÈRES SUPERPARAMAGNÉTIQUES ET HAUTEMENT POREUSES POUR DES APPLICATIONS DIAGNOSTIQUES

(30) Priority: 13.04.2017 EP 17166491
(43) Date of publication of application: 19.02.2020
(60) Divisional application: 26153703.9
(73) Proprietor: Roche Diagnostics GmbH, 68305 Mannheim (DE); F. Hoffmann-La Roche AG, 4070 Basel (CH)
(72) Inventor: BOLLE, Jens Christian, 82377 Penzberg (DE); SILVESTRE, Martin Eduardo, 82377 Penzberg (DE); HUG, Stephan, 82377 Penzberg (DE); BYLDA, Caroline, 82377 Penzberg (DE)
(74) Representative: Altmann Stößel Dick Patentanwälte PartG mbB
(86) International application number: PCT/EP2018/059383
(87) International publication number: WO 2018/189286

(56) References cited:
- WO-A1-84/02031
- US-A1- 2001 014 468
- US-A1- 2010 129 794
- US-B1- 6 503 762
- PETR S�LEK ET AL: "Immunomagnetic sulfonated hypercrosslinked polystyrene microspheres for electrochemical detection of proteins", JOURNAL OF MATERIALS CHEMISTRY, vol. 21, no. 38, 1 January 2011 (2011-01-01), GB, pages 14783, XP055758420, ISSN: 0959-9428, DOI: 10.1039/c1jm12475g
- ZHIFANG JIA ET AL: "Functionalized hypercrosslinked polymers with knitted N-heterocyclic carbene-copper complexes as efficient and recyclable catalysts for organic transformations", CATALYSIS SCIENCE & TECHNOLOGY, vol. 6, no. 12, 1 January 2016 (2016-01-01), UK, pages 4345 - 4355, XP055758422, ISSN: 2044-4753, DOI: 10.1039/C5CY02260F
- JANA KOUBKOV� ET AL: "Magnetic poly(glycidyl methacrylate) microspheres for protein capture", NEW BIOTECHNOLOGY, vol. 31, no. 5, 1 September 2014 (2014-09-01), NL, pages 482 - 491, XP055532714, ISSN: 1871-6784, DOI: 10.1016/j.nbt.2014.06.004
- ZHOU QING ET AL: "Reusable magnetic microspheres for efficient removal of atrazine in aqueous media", CHEMICAL ENGENEERING JOURNAL, vol. 253, 1 October 2014 (2014-10-01), AMSTERDAM, NL, pages 190 - 197, XP055869805, ISSN: 1385-8947, DOI: 10.1016/j.cej.2014.05.035
- ZHANG MANCHENG ET AL: "A magnetic sorbent for the efficient and rapid extraction of organic micropollutants from large-volume environmental water samples", JOURNAL OF CHROMATOGRAPHY A, ELSEVIER, AMSTERDAM, NL, vol. 1316, 2 October 2013 (2013-10-02), pages 44 - 52, XP028744787, ISSN: 0021-9673, DOI: 10.1016/J.CHROMA.2013.09.086
- WANG WEI ET AL: "Preparation of a permanent magnetic hypercrosslinked resin and assessment of its ability to remove organic micropollutants from drinking water", FRONTIERS OF ENVIRONMENTAL SCIENCE & ENGINEERING, HIGHER EDUCATION PRESS, HEIDELBERG, vol. 9, no. 1, 24 June 2014 (2014-06-24), pages 96 - 104, XP035423753, ISSN: 2095-2201, [retrieved on 20140624], DOI: 10.1007/S11783-014-0724-3
- MA YAN ET AL: "Preparation of a novel magnetic microporous adsorbent and its adsorption behavior ofp-nitrophenol and chlorotetracycline", JOURNAL OF HAZARDOUS MATERIALS, ELSEVIER, AMSTERDAM, NL, vol. 266, 15 December 2013 (2013-12-15), pages 84 - 93, XP028826930, ISSN: 0304-3894, DOI: 10.1016/J.JHAZMAT.2013.12.015
- LIANGXIAO TAN ET AL: "Hypercrosslinked porous polymer materials: design, synthesis, and applications", CHEMICAL SOCIETY REVIEWS, vol. 46, no. 11, 22 February 2017 (2017-02-22), pages 3322 - 3356, XP055406627, ISSN: 0306-0012, DOI: 10.1039/C6CS00851H
- TOLMACHEVA V V ET AL: "Magnetic adsorbents based on iron oxide nanoparticles for the extraction and preconcentration of organic compounds", JOURNAL OF ANALYTICAL CHEMISTRY, CONSULTANTS BUREAU. NEW YORK, US, vol. 71, no. 4, 7 April 2016 (2016-04-07), pages 321 - 338, XP035700960, ISSN: 1061-9348, [retrieved on 20160407], DOI: 10.1134/S1061934816040079

## Description

### Field of the invention

The present invention relates to magnetic particles, wherein each particle comprises a polymer matrix (P) and at least one magnetic core (M), wherein the polymer matrix comprises a hypercrosslinked polymer and wherein the magnetic particle has a particle size in the range of from 5 to 40 micrometers, as determined according to ISO 13320;
wherein the polymer matrix (P) comprises a co-polymer obtained or obtainable by a method comprising co-polymerizing suitable monomeric building blocks in the presence of one monomeric building block which is a crosslinking agent, wherein 5-90 vol% of all monomeric building blocks are divinylbenzenes;
wherein in the co-polymerization a polymer with a crosslinking degree of at least 5% is obtained;
wherein divinylbenzene is the crosslinking agentwherein the suitable monomeric building blocks are selected from the group consisting of styrene, functionalized styrenes, vinylbenzylchloride, divinylbenzene, vinylacetate, methylmethaacrylate and acrylic acid;
wherein the at least one magnetic core (M) comprises at least one iron oxide nanoparticle; wherein the at least one magnetic core (M) comprises at least one magnetic nanoparticle and a coating C1;
wherein the coating C1 is tenside or silica coating.

Further, the present invention relates to a method of preparing such particles the method comprising:
(i) providing at least one magnetic core (M), preferably at least two magnetic cores (M),
(ii) providing polymer precursor molecules,
(iii) polymerizing the polymer precursor molecules according to (ii) in the presence of the at least one magnetic core (M), thereby forming a particle comprising the at least one magnetic core (M), preferably the at least two magnetic cores (M), embedded in a polymer matrix (P1), wherein the polymer matric (P1) preferably comprises, more preferably consists of, a crosslinked polymer, and
(iv) hypercrosslinking the polymer matrix (P1) of the polymer particle obtained in (iii), to give the magnetic particle;

wherein the reaction in (iv) is not carried out in a solvent comprising dichloroethane or other organic halides;
wherein in (iii) the polymer matrix (P1) comprises a crosslinked polymer being obtained or obtainable by crosslinking a polymer with 5-90 vol% of a divinylbenzene crosslinking agent, based on the total amount of the polymer, wherein in the resulting polymer a crosslinking degree of at least 5% is obtained;
wherein divinylbenzene is the crosslinking agent;
wherein the hypercrosslinking, in (iv) is carried out at a temperature in the range of from -30 to 120°C;
wherein the reaction in (iv) is carried out in a solvent comprising at least THF, acetonitrile, DMF, dioxane or toluol.

Furthermore, the present invention relates to particles obtainable or obtained by said method. Moreover, the present invention is concerned with the use of these magnetic particles for qualitative and/or quantitative determination of at least one analyte in a fluid. Further, the present invention relates to a method for determining at least one analyte in a fluid sample comprising the contacting of a magnetic particle of the invention or a magnetic particle obtained by the method of the present invention with a fluid sample comprising or suspected to comprise the at least one analyte.

### Related art

Magnetic particles are a great tool for capturing analytes from human samples. When e.g. covered with antibodies, these particles are able to specifically capture analytes which can be detected by optical techniques. The magnetic properties are of great importance as they allow easy, fast and cheap automation on diagnostic systems and additionally avoid time-consuming centrifugation and filtration steps. Here, superparamagnetic materials get more attention as they only show magnetization when an external magnetic field is applied. In the absence of an external magnetic field, magnetization appears to be zero (no "memory effect"). E.g. EP2003455A1 and EP2015074A1 describe the extraction of analytes from human samples by using magnetic particles on an LC/MS system.

High specific surface areas on the magnetic particles are required to enrich analytes from human samples. To increase the surface area to more than 1000 m²/g, magnetic particles need to be coated with a porous matrix. This is usually done by embedding magnetic particles into a porous silica- or titanium oxide-matrix. One drawback is the high density of silica and titanium that leads to a decrease of magnetization with increasing film thickness. Furthermore, only mesoporous (pores > 2 nm) systems can be developed by using silica or titanium oxide but especially for small analytes, materials with micropores (pores < 2 nm) are preferred. Additionally, proteins and phospholipids are adsorbed into the large mesopores, which generates problematic matrix-effects in the LC/MS system.

One key requirement for the automation of the particles inside the enrichment-workflow-MS technology is a fast magnetic separation (<5 s) for high throughput. Particle size and saturation magnetization are crucial properties. Therefore particles with high saturation magnetization (>5 A m² kg⁻¹) and large sizes (>2 µm) are required. Additionally, for the robustness of the system, carry-over of particles has to be avoided. Therefore the particles need to have high magnetization and particle sizes larger than 1µm.

Particles having a polymer matrix with micropores are e.g. in Yang et al. Polym. Chem., 2013, 4, 1425. According to Yang *et al.* iron oxide nanoparticles are first coated with oleic acid and are afterwards embedded into a polystyrene-matrix via a mini-emulsion polymerization. To reach a high specific surface area, the nanoparticles are finally hypercrosslinked by a Friedel-Crafts reaction, making use of FeCl₃ as a catalyst and dimethoxymethane as porosity dependent crosslinking reagent. The resulting particles have a size of 100 nm in average and a saturation magnetization of 4.1 A m² kg⁻¹. The particles are described as being useful for extracting organic molecules from water and also as drug carriers to control ibu-profen drug delivery, however, they display only a relatively small particle size as well as a low saturation magnetization which makes them disadvantageous for automation processes.

Xu *et al.* describes the synthesis of citrate-stabilized iron oxide nanoparticles with grain sizes of about 300 nm (S. Xu et al. Polym. Chem., 2015, 6, 2892). These citrate-stabilized nanoparticles are coated with 3-(trimethoxysilyl) propyl methacrylate (MEMO) and covered with a polystyrol shell via a *soap-free* emulsion polymerization. With this technique, one nanoparticle gets embedded into the polymer particle. In a second polymerization, another layer is formed on the particle. This polymerization is called a *seeded swelling* polymerization and contains a different monomer composition. As a last step, porosity is formed by a hypercrosslinking reaction with FeCl₃ as pore-forming catalyst. However, with this process only sizes of about 400 nm are reached with a saturation magnetization of 14.1 A m² kg⁻¹.

WANG WEI ET AL: "Preparation of a permanent magnetic hypercrosslinked resin and assessment of its ability to remove organic micropollutants from drinking water", FRONTIERS OF ENVIRONMENTAL SCIENCE & ENGINEERING, HIGHER EDUCATION PRESS, HEIDELBERG, vol. 9, no. 1, 24 June 2014, pages 96-104, discloses a method based on a permanent magnetic hypercrosslinked resin Wl50 (prepared by suspension and post-crosslinking reaction) - for the removal of organic micropollutants in drinking water.

MA YAN ET AL: "Preparation of a novel magnetic microporous adsorbent and its adsorption behavior of p-nitrophenol and chlorotetracycline", JOURNAL OF HAZARDOUS MATERIALS, vol. 266, 15 December 2013, pages 84-93, discloses a method for fabricating hypercrosslinked magnetic polymer beads with improved acid resistance. Disclosed is that magnetite nanoparticles were covered with tetraethoxysilane and vinyltriethoxysilane, followed by co-polymerization and post-crosslinking.

LIANGXIAO TAN ET AL: "Hypercrosslinkedporous polymer materials: design, synthesis, and applications", CHEMICAL SOCIETY REVIEWS, vol. 46, no. 11, 22 February 2017, pages 3322-3356, discloses synthetic principles and strategies of hypercrosslinked polymers and advancements in the structural and morphological study as well as potential applications.

TOLMACHEVA V VET AL: "Magnetic adsorbents based on iron oxide nanoparticles for the extraction and preconcentration of organic compounds", JOURNAL OF ANALYTICAL CHEMISTRY, vol. 71, no. 4, 7 April 2016, pages 321-338, discloses approaches and methods used in the synthesis of magnetic adsorbents based on iron oxide nanoparticles. A classification of magnetic adsorbents is disclosed; examples of their use in the magnetic solid-phase extraction of organic compounds in the analysis of environmental samples, food, and biological fluids.

US 6 503 762 B1 discloses a magnetic carrier which comprises particulate silica containing a magnetic material, having polyacrylamide on the surface thereof in an amount ranging from 0.3 to 5 mmol/g in terms of monomeric acrylamide. Also disclosed is a process for producing the magnetic carrier in which the surface of particulate silica containing a magnetic material is treated with a coupling agent, and then treated particulate silica is reacted with acrylamide and/or polyacrylamide.

US 2001/014468 A1 discloses, magnetic, spherical polyvinyl alcohol (PVAL) particles produced through suspension of a polymer phase containing magnetic colloids in a vegetable oil phase containing a special emulsifier mixture.

US 2010/129794 A1 discloses, magnetic polymer particles including magnetic particles selected from the group of ferromagnetic, ferrimagnetic and/or superparamagnetic particles, where the magnetic particles are embedded in a crosslinked polyacrylate or polyalkylacrylate matrix

WO 84/02031 A1 discloses, magnetic polymer particles prepared by treating compact or porous polymer particles with a solution of iron salts and, if desired, salts of other metals which are capable of forming magnetic ferrites, in which the solution swells or penetrates into the particles.

Petr Sálek ET AL: "lmmunomagnetic sulfonated hypercrosslinked polystyrene microspheres for electrochemical detection of proteins", Journal of Materials Chemistry, vol. 21, no. 38, 2011, pages 14783-14792, discloses poly(styrene-co-divinylbenzene) microspheres of narrow size distribution prepared by (2-hydroxypropyl)cellulose-stabilized dispersion copolymerization of styrene and divinylbenzene in a 2-methoxyethanol/ethanol mixture under continuous addition of divinylbenzene. Disclosed is that the copolymerization was initiated with dibenzoyl peroxide and that the obtained microspheres were chloromethylated using several chloromethylation agents and then hypercrosslinked.

Zhifang Jia ET AL: "Functionalized hypercrosslinked polymers with knitted N-heterocyclic carbene-copper complexes as efficient and recyclable catalysts for organic transformations", Catalysis Science & Technology, vol. 6, no. 12, 2016, pages 4345-4355 discloses, an N-heterocyclic carbene (NHC)-copper complex supported on hypercrosslinked polymers (HCPs) synthesized through an external cross-linking reaction.

Jana Koubková ET AL: "Magnetic poly(glycidyl methacrylate) microspheres for protein capture", New BIOTECHNOLOGY, vol. 31, no. 5, September 2014, pages 482-491 discloses, superparamagnetic polymer microspheres for the specific isolation of the tumor suppressor protein p53. Disclosed is that monodisperse macroporous poly(glycidyl methacrylate) (PGMA) microspheres measuring approximately 5 µm and containing carboxyl groups were prepared by multistep swelling polymerization of glycidyl methacrylate (GMA), 2-[(methoxycarbonyl)methoxy]ethyl methacrylate (MCMEMA) and ethylene dimethylacrylate (EDMA) as a crosslinker in the presence of cyclohexyl acetate as a porogen. Disclosed is that to render the microspheres magnetic, iron oxide was precipitated within their pores.

Zhou Qing ET AL: "Reusable magnetic microspheres for efficient removal of atrazine in aqueous media", Chemical Engineering Journal, vol. 253, 1 October 2014, pages 190-197 discloses, magnetic hypercrosslinked microsphere (Q150) prepared and used for organic micropollutants (OMPs) removal. Disclosed is that Q150 has a high surface area (1103.6 m²/g) with a narrow particle size distribution (10-30 µm).

ZHANG MANCHENG ET AL: "A magnetic sorbent for the efficient and rapid extraction of organic micropollutants from large-volume environmental water samples", JOURNAL OF CHROMATOGRAPHY A, vol. 1316, 2 October 2013, pages 44-52 discloses, magnetic hypercrosslinked microspheres (NAND-1) prepared via membrane emulsification-suspension polymerization and post crosslinking reaction. Disclosed is that to ensure that the Fe₃O₄ nanoparticles could completely pass through the membrane without blocking the pores, oleic acid was used to modify the Fe₃O₄ nanoparticles, which enhanced lipophilicity and mono-dispersity of the magnetite nanoparticles. In this regard, further disclosed is a uniform particle size ( ~8 µm) and a large average surface area (1303.59 m² /g).

Thus, there is still the need for advantageous particles, in particular for particles which are useful for automation processes.

### Summary of the invention

It is therefore an object of the present invention to provide porous magnetic particles with a relatively large particle size as well as a relatively high saturation magnetization. This problem is solved by the invention with the features of the independent patent claims. Advantageous developments of the invention, which can be realized individually or in combination, are presented in the dependent claims and/or in the following specification and detailed embodiments.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once, typically will be used only once when introducing the respective feature or element. In the following, in most cases, when referring to the respective feature or element, the expressions "at least one" or "one or more" will not be repeated, notwithstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such a way with other optional or non-optional features of the invention.

In a first aspect of the present invention, a magnetic particle comprising a polymer matrix (P) and at least one magnetic core (M) is defined, wherein the polymer matrix comprises a hypercrosslinked polymer and wherein the magnetic particle has a particle size in the range of from 5 to 40 micrometers, as determined according to ISO 13320;
wherein the polymer matrix (P) comprises a co-polymer obtained or obtainable by a method comprising co-polymerizing suitable monomeric building blocks in the presence of one monomeric building block which is a crosslinking agent, wherein 5-90 vol% of all monomeric building blocks are divinylbenzenes;
wherein in the co-polymerization a polymer with a crosslinking degree of at least 5% is obtained;
wherein divinylbenzene is the crosslinking agentwherein the suitable monomeric building blocks are selected from the group consisting of styrene, functionalized styrenes, vinylbenzylchloride, divinylbenzene, vinylacetate, methylmethaacrylate and acrylic acid;
wherein the at least one magnetic core (M) comprises at least one iron oxide nanoparticle; wherein the at least one magnetic core (M) comprises at least one magnetic nanoparticle and a coating C1;
wherein the coating C1 is tenside or silica coating.

In a further aspect, the present invention relates to a method of preparing a magnetic particle comprising a polymer matrix (P) and at least one magnetic core (M), and to a magnetic particle obtained or obtainable by said method, wherein the polymer matrix (P) comprises at least one crosslinked polymer, wherein the magnetic particle has a particle size in the range of from 5 to 40 micrometers, as determined according to ISO 13320, the method comprising:
(i) providing at least one magnetic core (M) , preferably at least two magnetic cores (M),
(ii) providing polymer precursor molecules,
(iii) polymerizing the polymer precursor molecules according to (ii) in the presence of the at least one magnetic core (M), thereby forming a particle comprising the at least one magnetic core (M), preferably the at least two magnetic cores (M), embedded in a polymer matrix (P1), and
(iv) hypercrosslinking the polymer matrix (P1) of the polymer particle obtained in (iii),

to give the magnetic particle;
wherein the reaction in (iv) is not carried out in a solvent comprising dichloroethane or other organic halides;
wherein in (iii) the polymer matrix (P1) comprises a crosslinked polymer being obtained or obtainable by crosslinking a polymer with 5-90 vol% of a divinylbenzene crosslinking agent, based on the total amount of the polymer, wherein in the resulting polymer a crosslinking degree of at least 5% is obtained;
wherein divinylbenzene is the crosslinking agent;
wherein the hypercrosslinking, in (iv) is carried out at a temperature in the range of from -30 to 120°C;
wherein the reaction in (iv) is carried out in a solvent comprising at least THF, acetonitrile, DMF, dioxane or toluol.

In a third aspect, the present invention relates to the use of the magnetic particle described above and below or the magnetic particle obtainable by the method described above and below, for qualitative and/or quantitative determination of at least one analyte in a fluid.

Further, the present invention relates to a method for determining at least one analyte in a fluid sample comprising the contacting of a magnetic particle of the invention or a magnetic particle obtained by the method of the present invention with a fluid sample comprising or suspected to comprise the at least one analyte.

### The magnetic particle

The magnetic particles according to the invention have a particle size in the range of from 5 to 40 micrometers, as determined according to ISO 13320. Preferably, the particle size is in the range of from 20 to 40 micrometers, more preferably, the particle size is in the range of from 20 to 35 micrometers.

As described above, the magnetic particle according to the invention comprises a polymer matrix (P) and at least one magnetic core (M). According to a preferred embodiment of the invention, the magnetic particle comprises more than one magnetic core (M), i.e. each particle preferably comprises at least one and, preferably, at least two magnetic cores (M). The magnetic core (M) comprises one or more magnetic nanoparticles, such as e.g. 1 to 20 magnetic nanoparticles, preferably 1 to 10, more preferably, 1 to 5 and most preferably 1 to 3 magnetic nanoparticles. Alternatively, it may comprise more than 20 nanoparticles and, preferably 100 to 1.5 million nanoparticles more preferably 750 -750,000 nanoparticles, more preferably 1,750 - 320,000 nanoparticles, in particular 90,000 - 320,000 nanoparticles.

Thus, the present invention also relates to a magnetic particle as described above, as well as to a magnetic particle obtained or obtainable by the above-described method, wherein the particle comprises at least two magnetic cores (M). According to a particularly preferred embodiment, the magnetic particle, as well as to a magnetic particle obtained or obtainable by the above described method, consists of the at least two magnetic cores (M) and the polymer matrix (P).

Preferably the amount of magnetic cores (M) is chosen so that a desired saturation magnetization saturation of the final particle is achieved. Preferably, the magnetic particle according to the invention, or the particle obtained or obtainable by the above-described method, has a saturation magnetization of at least 1 A m²/kg. Preferably, the saturation magnetization is at least 1 A m²/kg, more preferably at least 2 A m²/kg, more preferably at least 3 A m²/kg, more preferably at least 4 A m²/kg, more preferably at least 5 A m²/kg , more preferably at least 6 A m²/kg, more preferably at least 7 A m²/kg, more preferably at least 8 A m²/kg, more preferably at least 9 A m²/kg , and in particular at least 10 A m²/kg, such as in the range of from 10 A m²/kg to 20 A m²/kg, more preferably in the range of from 10 A m²/kg to 30 A m²/kg, as determined according to ASTM A 894/A 894M.

The particle of the present invention may, in principle, display any geometrical form, however, preferably, the particle is substantially spherical. As used herein, the term "substantially spherical" refers to particles with rounded shapes that are preferably non-faceted or substantially free of sharp corners. In certain embodiments, the substantially spherical particles typically have an average aspect ratio of less than 3:1 or 2:1, for example, an aspect ratio less than 1.5:1, or less than 1.2:1. In a certain embodiment, substantially spherical particles may have an aspect ratio of about 1:1. The aspect ratio (A_{R}) is defined as being a function of the largest diameter (dₘₐₓ) and the smallest diameter (dₘᵢₙ) orthogonal to it (A_{R} = dₘᵢₙ/dₘₐₓ). The diameters are determined via SEM or light microscope measurements.

The BET specific surface area of the magnetic particle as described above, as well as to a magnetic particle obtained or obtainable by the above-described method, is preferably in the range of from 50 to 2500 m²/g, as determined according to ISO 9277. More preferably, the BET specific surface area of the magnetic particle is in the range of from 100 to 1500 m²/g and in particular in the range of from 300 to 1000 m²/g

According to a preferred embodiment of the present invention, the magnetic particle as described above, as well as to a magnetic particle obtained or obtainable by the above described method, is superparamagnetic. The term "superparamagnetic" is known to the person skilled in the art and refers to the magnetic property encountered in particular for particles smaller than a single magnetic mono-domain. Such particles steadily orient upon applying an external magnetic field until a maximum value of the global magnetization, dubbed saturation magnetization, is reached. They relax when removing the magnetic field, with no magnetic hysteresis (no remanence) at room temperature. In the absence of an external magnetic field, superparamagnetic particles exhibit a non-permanent magnetic moment due to thermal fluctuations of the dipole orientation (Neel relaxation) and particle position (Brownian relaxation).

### The magnetic core (M)

As described above, the magnetic particles according to the invention comprise at least one magnetic core (M) and preferably at least two magnetic cores (M). Preferably, the at least one magnetic core (M) comprises a compound selected from the group consisting of metal, metal carbide, metal nitride, metal sulfide, metal phosphide, metal chelate and a mixture of two or more thereof. The at least one magnetic core (M) may also comprise an alloy with a metal such as gold, silver, platinum, or copper.

It is to be understood that each magnetic core (M) may comprise a mixture of two or more of the above-mentioned group, i.e. two or more of a metal, metal carbide, metal nitride, metal sulfide, metal phosphide, a metal chelate and a mixture of two or more thereof. Further, mixtures of two or more different metals, two or more different metal oxides, two or more different metal carbides, two or more different metal nitrides, two or more different metal sulphides, two or more different metal phosphides, two or more different metal chelates are conceivable.

Further, it is to be understood that in case the magnetic particle according to the invention comprises more than one magnetic core (M), each of the magnetic cores (M) present in a single particle may be the same or may differ from each other. Preferably, all magnetic cores (M) comprised in one magnetic particle are the same.

More preferably, the at least one magnetic core (M) comprises a metal carbide.

In a preferred embodiment, the at least one magnetic core (M) comprises a metal, metal carbide, metal nitride, metal sulfide, metal phosphide, or metal chelate comprising at least one transition metal. Preferred transition metals according to the invention include, but are not limited to, chromium, manganese, iron, cobalt, nickel, zinc, cadmium, nickel, gadolinium, copper, and molybdenum. More preferably, the metal, metal carbide, metal nitride, metal sulfide, metal phosphide,or metal chelate comprises at least iron. More preferably, the at least one magnetic core (M) comprises an iron oxide selected from the group consisting of Fe₃O₄, α-Fe₂O₃, γ- Fe₂O₃, MnFeₓO_{y}, CoFeₓO_{y}, NiFeₓO_{y}, CuFeₓO_{y}, ZnFeₓO_{y},, CdFeₓO_{y}, BaFeₓO and SrFeₓO, wherein x and y vary depending on the method of synthesis, and wherein x is preferably an integer of from 1 to 3, more preferably 2, and wherein y is preferably 3 or 4. Most preferably, the at least one magnetic core (M) comprises Fe₃O₄.

Thus, the present invention relates to a magnetic particle as described above, as well as to a magnetic particle obtained or obtainable by the above-described method, wherein the at least one magnetic core (M) comprises an iron oxide. Most preferably, the at least one magnetic core (M) comprises Fe₃O₄.

The magnetic core (M) comprises at least one nanoparticles and a coating C1, wherein the coating C1 is tenside or silica coating.

### Nanoparticles

The Nanoparticles are preferably the part which displays the magnetism, preferably super-paramagnetism of a particle. Nanoparticles are sometimes also referred to as "magnetic nanoparticles" herein.

Preferably, the at least one nanoparticle comprises, preferably consists of, at least one magnetic, preferably superparamagnetic, nanoparticle and optionally one coating, such as a coating C2.

As used herein, the term "nanoparticle" refers to a particle being less than 100 nanometers in at least one dimension, i.e. having a diameter of less than 100 nm. Preferably, the nanoparticle according to the invention has a diameter in the range of from 1 to 20 nm, preferably 4 to 15 nm, as determined according to TEM-measurements. Thus, according to a preferred embodiment, the present invention also relates to a magnetic particle as described above, as well as to a magnetic particle obtained or obtainable by the above-described method, wherein the magnetic particle comprises at least one magnetic core (M) which comprises at least one nanoparticle and optionally one coating, such as a coating C2.

Each nanoparticle(s), preferably has/have a diameter in the range of from 1 to 20 nm, preferably 4 to 15 nm, as determined according to TEM-measurements. Preferably, the at least one magnetic nanoparticle is superparamagnetic.

The magnetic core (M) may comprise only one nanoparticle or more than one nanoparticle. In one embodiment, it comprises from 1 to 20 nanoparticles. In another embodiment, it comprises 100 to 1.5 million nanoparticles more preferably 750 -750,000 nanoparticles, more preferably 1,750 - 320,000 nanoparticles, in particular 90,000 - 320,000 nanoparticles. The nanoparticles may be present as magnetic core in the form of individual (i.e. separate) particles or they may for aggregates consisting of several nanoparticles. Theses aggregates may have different sizes depending on the number of included nanoparticles. Typically, so called supraparticles are formed, which are described further below in more detail. In the case of a magnetic core comprising 100 or more nanoparticles, the nanoparticles typically form such supraparticles.

### A magnetic core (M) comprising 1-20 nanoparticles

According to a first embodiment, the magnetic core (M) comprises, preferably consists of, 1-20 magnetic nanoparticles and optionally a coating C2, i.e. one magnetic nanoparticle, optionally with the coating C2, forms the nanoparticle of the magnetic core (M). Typically, the magnetic core comprises 1 to 20 magnetic nanoparticles, preferably 1 to 10, more preferably, 1 to 5 and most preferably 1 to 3 nanoparticles.

Preferably, in this case, the nanoparticle, comprises, more preferably consists of a compound selected from the group consisting of metal, metal carbide, metal nitride, metal sulfide, metal phosphide, metal oxide, metal chelate and a mixture of two or more thereof. It is to be understood that each nanoparticle may comprise, preferably consist of, a mixture of two or more of the above mentioned group, i.e. two or more of a metal, metal carbide, metal nitride, metal sulfide, metal phosphide, metal oxide, metal chelate and a mixture of two or more thereof. Further, mixtures of two or more different metals, two or more different metal oxides, two or more different metal carbides, two or more different metal nitrides, two or more different metal sulphides, two or more different metal chelates or two or more different metal phosphides are conceivable. More preferably, the nanoparticle comprises, more preferably consists of a metal oxide or a metal carbide. In a preferred embodiment, the metal is a transition metal. Preferred transition metals according to the invention include, but are not limited to, chromium, manganese, iron, cobalt, nickel, zinc, cadmium, nickel, gadolinium, copper, and molybdenum. Most preferably, the metal is iron.

Thus, the nanoparticle comprises, more preferably consists of iron oxide, preferably Fe₃O₄.

According to this embodiment, it is preferred that in case more than one magnetic cores (M) are present in the magnetic particle, these magnetic cores (M) are not aggregated with each other. Preferably, these particles are substantially evenly distributed within the polymer matrix.

### A magnetic core (M) comprising a supraparticle

According to a second preferred embodiment, the magnetic core (M) comprises more than 20 nanoparticles, and, typically more than 100 nanoparticles, wherein these nanoparticles are preferably aggregated with each other to form a supraparticle. More preferably, in this case, the magnetic core (M) comprises a supraparticle consisting of aggregated, coated, nanoparticles. Preferably, in this case, the magnetic core (M) comprises a supraparticle which comprises between 100 to 1.5 million nanoparticles more preferably 750 -750,000 nanoparticles, more preferably 1,750 - 320,000 nanoparticles, in particular 90,000 - 320,000 nanoparticles. Preferably, each nanoparticle is coated with at least one coating C2. Preferably in this case, the magnetic core (M) thus comprises, preferably consists of, the supraparticle, which consist of, coated, nanoparticles being aggregated with each other, wherein the nanoparticles are coated with at least one coating C2, and wherein the coating is preferably deposited on the surface of the nanoparticles. The supraparticle may preferably also be coated with a coating C1.

Thus according to this second preferred embodiment of the invention, the magnetic particle according to the invention comprises more than 20 magnetic nanoparticles, and preferably 100 to 1.5 million nanoparticles, wherein said nanoparticles form at least one supraparticle. Each of the nanoparticles in the supraparticle is typically coated with at least one coating C2 and the supraparticle is typically coated with at least one coating C1.

Preferably, the coating C2 is a coating which covers at least a part, preferably the whole surface, of each nanoparticle. Preferably, also in this case, each nanoparticle comprises, more preferably consists of, a compound selected from the group consisting of metal, metal carbide, metal nitride, metal sulfide, metal phosphide, metal oxide, metal chelate and a mixture of two or more thereof. It is to be understood that each nanoparticle present in the supraparticle may comprise, preferably consist of, a mixture of two or more of the above-mentioned group, i.e. two or more of a metal, metal carbide, metal nitride, metal sulfide, metal phosphide, metal oxide, metal chelate and a mixture of two or more thereof. Further, mixtures of two or more different metals, two or more different metal oxides, two or more different metal carbides, two or more different metal nitrides, two or more different metal sulphides, two or more different metal chelates or two or more different metal phosphides are conceivable. More preferably, each nanoparticle in the supraparticle comprises, more preferably consists of, a metal oxide or a metal carbide. In a preferred embodiment, the metal is a transition metal. Preferred transition metals according to the invention include, but are not limited to, chromium, manganese, iron, cobalt, nickel, zinc, cadmium, nickel, gadolinium, copper, and molybdenum. Most preferably, the metal is iron. According to a particularly preferred embodiment, each nanoparticle comprised in the supraparticle is a metal oxide nanoparticle, most preferably an iron oxide nanoparticle, in particular a Fe₃O₄-nanoparticle.

Thus, the present invention also relates to a magnetic particle as described above, as well as to a magnetic particle obtained or obtainable by the above described method, wherein the magnetic core (M) comprises or preferably consists of a supraparticle consisting of aggregated at least 20 magnetic nanoparticles wherein the nanoparticles are preferably being coated with at least one coating C2.

Preferably, the magnetic core (M) including the at least one coating C1, wherein the coating C1 is tenside or silica coating has a diameter in the range of from 80 to 500 nm, more preferably 150 to 400 nm, and most preferably 200 to 300 nm, as determined according to DLS (ISO 22412).

### The coating C2

As coating C2, in general any coating known to those skilled in the art is conceivable. Preferably, the coating C2 is, however, selected from at least one member of the group consisting of dicarboxylic acids, tricarboxylic acids, polyacrylic acid, amino acids, surfactants and fatty acids. It is to thus be understood that the aforementioned group includes salts and derivatives, such as esters and polymers, of the mentioned compounds. Thus, the coating C2 is preferably selected from at least one member of the group consisting of dicarboxylic acids, dicarboxylic acid salts, dicarboxylic acid derivatives, tricarboxylic acids, tricarboxylic acid salts, tricarboxylic derivatives, polyacrylic acid, polyacrylic acid salts, polyacrylic acid derivatives, amino acids, amino acid salts, amino acid derivatives, surfactants, salt of surfactants, fatty acids, fatty acid salts and fatty acid derivatives.

As used herein, the terms coated or coating are used to refer to the process of adsorption, van der Waals and/or non-polar group interactions (e.g., chemisorption or physical adsorption), or covalent binding of the magnetic nanoparticle or supraparticle core and the coating C2 or C1 or between two or more coatings, if present.

Preferably as fatty acids, fatty acid salts or fatty acid derivatives, such compounds are chosen which are capable of binding to the surface of the supraparticle, thereby preferably stabilizing the supraparticle. A fatty acid employed as coating C2 is preferably a single chain of alkyl groups containing from 8 to 22 carbon atoms with a terminal carboxyl group (-COOH) and high affinity adsorption (e.g., chemisorption or physical adsorption) to the surface of the magnetic particle. The fatty acid has multiple functions including protecting the magnetic particle core from oxidation and/or hydrolysis in the presence of water, which can significantly reduce the magnetization of the nanoparticle (Hutten, et al. (2004) J. Biotech. 112:47-63); stabilizing the nanoparticle core; and the like. The term "fatty acid" includes saturated or unsaturated, and in particular unsaturated fatty acids. Exemplary saturated fatty acids include lauric acid, myristic acid, palmitic acid, stearic acid, arachidic acid, propionic acid, butyric acid, valeric acid, caproic acid, enanthic acid, caprylic acid, pelargonic acid, capric acid, undecylic acid, tridecylic acid, pentadecylic acid, margaric acid, nonadecylic acid, heneicosylic acid, behenic acid, tricosylic acid, lignoceric acid, pentacosylic acid, cerotic acid, heptacosylic acid, montanic acid, nonacosylic acid, melissic acid, henatriacontylic acid, lacceroic acid, psyllic acid, geddic acid, ceroplastic acid, hexatriacontylic acid, hepta-triacontanoic acid and octatriacontanoic acid and the like. Exemplary unsaturated fatty acids include oleic acid, linoleic acid, linolenic acid, arachidonic acid, hexadecatrienoic acid, stea-ridonic acid, eicosatrienoic acid, eicosatetraenoic acid, eicosapentaenoic acid, heneicosapen-taenoic acid, docosapentaenoic acid, clupanodonic acid, docosahexaenoic acid, tetracosapentaenoic acid, tetracosahexaenoic acid, calendic acid, eicosadienoic acid, docosadienoic acid, adrenic acid, docosapentaenoic acid, tetracosatetraenoic acid, tetracosapentaenoic acid, 5-dodecenoic acid, 7-tetradecenoic acid, palmitoleic acid, vaccenic acid, paullinic acid, 15-docosenoic acid, 17-tetracosenoic acid, elaidic acid, gondoic acid, mead acid, erucic acid, nervonic acid, rumenic acid, calendic acid, jacaric acid, eleostearic acid, catalpic acid, punicic acid, rumelenic acid, parinaric acid, bosseopentaenoic acid, pinolenic acid, podocar-pic acid and the like. The fatty acid can be synthetic or isolated from a natural source using established methods. Moreover, a fatty acid can be a derivative such as a fatty acid enol ester (i.e., a fatty acid reacted with the enolic form of acetone), a fatty ester (i.e., a fatty acid with the active hydrogen replaced by the alkyl group of a monohydric alcohol), a fatty amine or fatty amide, or in particular embodiments, a fatty alcohol as described above. A particularly preferred fatty acid is oleic acid.

A surfactant, as used in the context of the instant invention, is an organic compound that is amphipathic, i.e., containing both hydrophobic groups and hydrophilic groups. Preferably surfactants are chosen which are capable of binding to the surface of the supraparticle thereby preferably stabilizing the supraparticle surfactants with a variety of chain lengths, hydrophilic-lipophilic balance (HLB) values and surfaces charges can be employed depending upon the application. Preferably, the surfactant according to the invention is a quateran-ary ammonium salt, alkylbenzenesulfonates, lignin sulfonates, polyoxylethoxylate, or sulfate ester. Non-limiting examples of surfactants are cetyltrimethylammonium bromide, cetyltrimethylammonium chloride, nonyphenolpolyethoxylates (i.e. NP-4, NP-40 and NP-7), sodium dodecylbenzenesulfonate, ammonium lauryl sulfate, sodium laureth sulfate, sodium myreth sulfate, docusate, perfluorooctanesulfonate, perfluorobutanesulfonate, alkylaryl ether phosphates, alkyl ether phosphates, sodium stearate, 2-Acrylamido-2-methylpropane sulfonic acid, ammonium perfluorononanoate, magnesium laureth sulfate, perfluoro-nonanoic acid, perfluorooctanoic acid, phospholipids, potassium lauryl sulfate, sodium alkyl sulfate, sodium dodecyl sulfate, sodium laurate, sodium lauroyl sarcosinate, sodium nona-noyloxybenzenesulfonate, sodium pareth sulfate, behentrimonium chloride, benzalkonium chloride, benzethonium chloride, bronidox, dimethyldioctadecylammonium bromide, dimethyldioctadecylammonium chloride, lauryl methyl gluceth-10 hydroxypropyl dimonium chloride, octenidine dihydrochloride, olaflur, N-oleyl-1,3-propanediamine, stearalkonium chloride, tetramethylammonium hydroxide, thonzonium bromide, cetomacrogol 1000, ce-tostearyl alcohol, cetyl alcohol, cocamide DEA, cocamide MEA, decyl polyglucose, disodium cocoamphodiacetate, glycerol monostearate, polyethylene glycol isocetyl ether, octylphenoxypolyethoxyethanol, lauryl glucoside, maltosides, monolaurin, mycosubtilin, non-oxynols, octaethylene glycol monododecyl ether, N-octyl beta-D-thioglucopyranoside, octyl glucoside, oleyl alcohol, pentaethylene glycol monododecyl ether, polidocanol, poloxamer, polyethoxylated tallow amine, polyglycerol polyricinoleate, polysorbate, sorbitan, sorbitan monolaurate, sorbitan monostearate, sorbitan tristearate, stearyl alcohol, surfactin, Triton X-100, Tween 80, cocamidopropyl betaine, cocamidopropyl hydroxysultaine, dipalmito-ylphosphatidylcholine, hydroxysultaine, lLauryldimethylamine oxide, lecithin, myristamine oxide, peptitergents, sodium lauroamphoacetate and bis(2-ethylhexyl)sulfosuccinic ester.

The term "amino acids" as used within the meaning of the present invention refers to natural or unnatural amino acids or amino acid derivatives as well as to salts of amino acids. Preferably, amino acids are chosen which are capable of binding to the surface of the supraparticle thereby preferably stabilizing the supraparticle. Exemplary amino acids include cysteine, methionine, histidine, alanine, arginine, asparagine, aspartic acid, glutamic acid, glutamine, glycine, isoleucine, leucine, lysine, phenylalanine, proline, serine, threonine, tryptophan, tyrosine, valine, selenocysteine, pyrrolysine, cysteine, dehydroalanine, enduracididine, lanthi-onine, norvaline and derivatives thereof.

The term "dicarboxylic acid" within the meaning of the present invention refers to a hydrocarbon or substituted hydrocarbon containing two carboxylic acid functional groups (i.e., R¹-(C(O)OH)₂), where R¹ is (a) a linear hydrocarbon containing from 0-18 carbon units or (b) a cyclic hydrocarbon containing 3- 8 carbon units, either as aromatic or non-aromatic rings. The term includes salts and derivatives of fatty acids, such as esters of fatty acids. Representative dicarboxylic acids are e.g. propanedioic acid, butanedioic acid, pentanedioic acid, hexanedioic acid, heptanedioic acid, octanedioic acid, nonanedioic acid, decanedioic acid, undecanedioic acid, dodecanedioic acid, hexadecanedioic acid, maleic acid, fumaric acid, glutaconic acid, traumatic acid, muconic acid, glutinic acid, citraconic acid, mesaconic acid, malic acid, aspartic acid, glutamic acid, tartronic acid, tartaric acid, diaminopimelic acid, saccharic acid, mesoxalic acid, oxaloacetic acid, acetonedicarboxylic acid, arabinaric acid, phthalic acid, isophthalic acid, terephthalic acid, diphenic acid, 2,6-naphthalenedicarboxylic acid.

The term "tricarboxylic acid" within the meaning of the present invention refers to a hydrocarbon or substituted hydrocarbon containing three carboxylic acid functional groups (i.e., R¹-(C(O)OH)₃), where R¹ is (a) a linear hydrocarbon containing from 3-18 carbon units or (b) a cyclic hydrocarbon containing 3- 8 carbon units, either as aromatic or non-aromatic rings. The term includes salts and derivatives of fatty acids, such as esters of fatty acids. Representative tricarboxylic acids are e.g. citric acid (2-hydroxypropane-1,2,3 tricarboxylic acid), isocitric acid (l-hydroxypropane-1,2,3 tricarboxylic acid), aconitic acid (prop-l-ene-1,2,3 tricarboxylic acid), propane-1,2,3-tricarboxylic acid, trimellitic acid (benzene-1,2,4-tricarboxylic acid), trimesic acid (benzene-l,3,5-tricarboxylic acid), oxalosuccinic acid (1-oxopropane-1,2,3-tricarboxylic acid) or hemimellitic acid (benzene-l,2,3-tricarboxylic acid). Preferably, the tricarboxylic acid is citric acid including citrates, i.e. salts and derivatives of citric acid.

Preferably, C2 is selected from the group consisting of citric acid, histidine, CTAB, CTAC, sodium oleate, polyacrylic acid or mixtures of two or more thereof (including the respective salts or derivatives thereof). Thus, the present invention also relates to a magnetic particle as described above, as well as to a magnetic particle obtained or obtainable by the above-described method, wherein the magnetic core (M) preferably consists of, a supraparticle consisting of aggregated magnetic nanoparticles with at least one coating C2, wherein the at least one coating C2 is selected from the group consisting of citrate, histidine, CTAB, CTAC, sodium oleate, polyacrylic acid or mixtures of two or more thereof.

Preferably the amount of coating C2 is in the range of from 1 to 80% by weight, more preferably in the range of from 5 to 70% by weight, more preferably in the range of from 10 to 50% by weight, most preferably 20 to 40% based on the total weight of the sum of C2 and the supraparticle.

### The coating C1

As described above, the magnetic core (M), comprises, more preferably consists of, magnetic nanoparticles and a coating C1, wherein the coating C1 is tenside or silica coating. Thus, the present invention also relates to a magnetic particle as described above, as well as to a magnetic particle obtained or obtainable by the above-described method, wherein the at least one magnetic core (M) comprises a coating C1.

The coating C1 is preferably deposited on the surface of the magnetic core (M). It is to be understood that between coating C1 and the magnetic core (M), further separating layers may exist, however, according to a preferred embodiment, C1 is coated directly on the magnetic core (M).

Preferably, the coating C1 surrounds the whole surface of the magnetic core (M).

Thus, the present invention relates to a magnetic particle as described above, as well as to a magnetic particle obtained or obtainable by the above-described method, comprising at least one magnetic core (M), wherein the at least one magnetic core (M) comprises at least one coating C1, wherein the coating C1 is tenside or silica coating.

Preferably, each magnetic core (M) comprises the coating C1 in an amount of from 1 to 40% by weight, preferably from 2 to 15% by weight, more preferably from 5 to 10% by weight, based on the total weight of at least one magnetic core (M).

### The polymer matrix (P)

As described above, each particle comprises besides the at least one magnetic core (M) a polymer matrix (P).

Preferably, the polymer matrix (P) is a porous polymer matrix, preferably a porous polymer matrix comprising pores having a pore size smaller than 100 nm, more preferably smaller than 100 nm, more preferably smaller than 90 nm, more preferably smaller than 80 nm, more preferably smaller than 70 nm, more preferably smaller than 60 nm, more preferably smaller or equal to 50 nm, such as in the range of from 0.5 nm to 50 nm, preferably in the range of from 1 to 20 nm as determined according to ISO 15901.

Thus, the present invention also relates to a magnetic particle as described above, as well as to a magnetic particle obtained or obtainable by the above-described method, wherein the polymer matrix (P) is a porous polymer matrix comprising pores having a pore size smaller than 100 nm, preferably smaller or equal to 50 nm, as determined according to ISO 15901.

Preferably at least 90% of all pores present in the polymer matrix have a pore size smaller than 10 nm and at least 50% of all pores present in the polymer matrix have a pore size smaller than 5 nm, as determined according to ISO 15901.

According to a particularly preferred embodiment, the polymer matrix does not comprise macropores, i.e. pores having a pore size larger than 50 nm.

Preferably, the particle comprises the polymer matrix (P) in an amount in the range of from 40 to 98% by weight, more preferably in the range of from 50 to 95% by weight, more preferably in the range of from 60 to 90% by weight, and most preferably in the range of from 70 to 85% by weight, based on the total weight of the particle.

The polymer matrix (P) comprises a co-polymer obtained or obtainable by a method comprising co-polymerizing suitable monomeric building blocks in the presence of one monomeric building block which is a crosslinking agent, wherein 5-90 vol% of all monomeric building blocks are divinylbenzenes;
wherein in the co-polymerization a polymer with a crosslinking degree of at least 5% is obtained;
wherein divinylbenzene is the crosslinking agentwherein the suitable monomeric building blocks are selected from the group consisting of styrene, functionalized styrenes, vinylbenzylchloride, divinylbenzene, vinylacetate, methylmethaacrylate and acrylic acid. Preferably, the co-polymer obtained or obtainable by a method comprising a polymerization of at least two different monomeric building blocks selected from the group consisting of the following monomers: 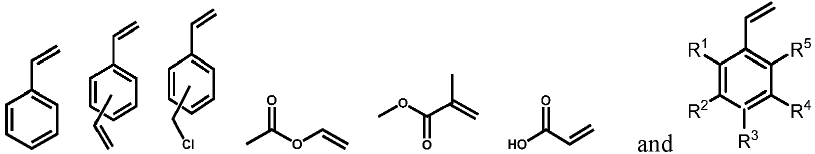
with R¹, R², R³, R⁴ and R⁵, are, independently of each other selected from the group consisting of -N₃, -NH₂, -Br, -I, -F, -NR'R'', -NR'R''R''', -COOH, -CN, -OH, -OR', -COOR', - NO₂, -SH₂, -SO₂, -R'(OH)ₓ, -R'(COOH)ₓ, -R'(COOR'')ₓ, -R'(OR")ₓ, -R'(NH2)ₓ, - R'(NHR")ₓ, -R'(NRʺR‴)ₓ, -R'(Cl)ₓ, -R'(I)ₓ, -R'(Br)ₓ, -R'(F)ₓ, R'(CN)ₓ, -R'(N₃)ₓ, - R'(NO₂)ₓ, -R'(SH₂)ₓ, -R'(SO₂)ₓ, alkyl, aryl, cycloalkyl, heteroaryl, heterocycloalkyl and with R', Rʺ and R‴ being, independently of each other, selected from the group consisting of alkyl, aryl, cycloalkyl, heteroaryl, heterocycloalkyl, halides, hydrogen, sulfides, nitrates and amines, and wherein x is an integer in the range of from 1 to 3.

As described above, divinylbenzene is employed as crosslinking agent.

As described above, the polymer matrix is obtained or obtainable by a method comprising co-polymerizing monomeric building blocks, wherein 5-90 vol% of all monomeric building blocks are divinylbenzenes,wherein a crosslinking degree of at least 5% is obtained.

The at least one magnetic core (M) is preferably embedded in the polymer matrix. The term "embedded" in this context is denoted to mean the magnetic core is preferably fully surrounded by the polymer matrix. Alternatively, it may be partially surrounded by the polymer matrix. In this case, the polymer matrix , however, immobilizes the magnetic core.

As described above, according to a preferred embodiment, the particle comprises at least two magnetic cores (M). In this case, it is to be understood, that each magnetic core (M) present in the particle is embedded in the polymer matrix (P). Thus, the present invention also relates to a magnetic particle as described above, as well as to a magnetic particle obtained or obtainable by the above-described method, wherein the at least two magnetic cores (M) are embedded in the polymer matrix.

### Hypercrosslinking

The polymer matrix comprises, in particular consists of a hypercrosslinked polymer.

The term "hypercrosslinked" as used herein refers to a type of multiple crosslinking resulting in a rigid three-dimensional network. Preferably, the hypercrosslinking is achieved by subjecting the crosslinked polymer to a chemical reaction, thereby obtaining the hypercrosslinked polymer. Thus, the polymer matrix (P) is a polymer matrix being obtained or obtainable by further hypercrosslinking the polymer with or without a hypercrosslinking agent by a chemical reaction. Suitable agents for hypercrosslinking polymers are known to those skilled in the art, and include, but are not limited to dichloroethane, dichloromethane, chloroform, carbon tetrachloride, dichlorobenzenes, trichlorobenzenes, dichloroalkanes, dibro-moalkanes, diiodoalkanes, trichloroalkanes, tribromoalkanes, triiodoalkanes, dimethoxymethane, dimethoxyethane and mixtures of two or more thereof.

Preferably, the hypercrosslinking is achieved by subjecting the crosslinked polymer matrix to a Friedel-Crafts reaction, in particular to a Friedel-Crafts reaction as described hereinunder.

### Surface functionalization

It is to be understood that the magnetic particles, comprising the at least one magnetic core M and the polymer matrix (P), may further comprise a surface modification. The surface of the particle, thus the surface of the polymer matrix (P) is preferably functionalized with at least one group selected from the group consisting of -OH, -COOH, diethylaminoethanol, R-SO₂-OH, -NH₂, R-SO₂-OH, -RNH, -R₂N, -R₃N⁺ -CH₃, -C₂H₅, -C₄H₉, -C₈H₁₇, -C₁₈H₃₇, - C₆H₅, -C₆H₉NO₆, Phenyl-Hexyl, Bi-Phenyl, Hydroxyapatit, boronic acid, biotin, azide, epoxide, alkyl, aryl, cycloalkyl, heteroaryl, heterocycloalkyl, aminoacids, -COOR, -COR, - OR, antibodies and fragments thereof, aptameres, nucleic acids, and receptor proteins or binding domains thereof. Preferably, these groups are being covalently attached to suitable functional groups of the polymer matrix. Ways to carry out such modifications are known to those skilled in the art.

### Method for preparing the magnetic particle

As described above, the present invention also relates to a method of preparing a magnetic particle comprising a polymer matrix (P) and at least one magnetic core (M), preferably at least two magnetic cores (M), wherein the polymer matrix (P) comprises at least one crosslinked polymer, wherein the magnetic particle has a particle size in the range of from 5 to 40 micrometers, the method comprising:
(i) providing at least one magnetic core (M), preferably at least two magnetic cores (M),
(ii) providing polymer precursor molecules,
(iii) polymerizing the polymer precursor molecules according to (ii) in the presence of the at least one magnetic core (M), thereby forming a particle comprising the at least one magnetic core (M), preferably the at least two magnetic cores (M), embedded in a polymer matrix (P1), wherein the polymer matric (P1) preferably comprises, more preferably consists of a crosslinked polymer, and
(iv) hypercrosslinking the polymer matrix (P1) of the particle obtained in (iii),

to give the magnetic particle;
wherein the reaction in (iv) is not carried out in a solvent comprising dichloroethane or other organic halides;
wherein in (iii) the polymer matrix (P1) comprises a crosslinked polymer being obtained or obtainable by crosslinking a polymer with 5-90 vol% of a divinylbenzene crosslinking agent, based on the total amount of the polymer, wherein in the resulting polymer a crosslinking degree of at least 5% is obtained;
wherein divinylbenzene is the crosslinking agent;
wherein the hypercrosslinking, in (iv) is carried out at a temperature in the range of from -30 to 120°C;
wherein the reaction in (iv) is carried out in a solvent comprising at least THF, acetonitrile, DMF, dioxane or toluol.

Preferably in (iii), the at least magnetic core (M) is embedded into the matrix.

### Step (i)

As described above, the at least one magnetic core (M) preferably comprises a compound selected from the group consisting of metal, metal carbide, metal nitride, metal sulfide, metal phosphide, metal oxide, metal chelate and a mixture of two or more thereof. The at least one magnetic core (M) may also comprise an alloy with a metal such as gold, silver, platinum, or copper. More preferably, the at least one magnetic core (M) comprises a metal oxide or a metal carbide, more preferably, the at least one magnetic core (M) comprises an iron oxide, in particular an iron oxide selected from the group consisting of Fe₃O₄, α-Fe₂O₃, γ- Fe₂O₃, MnFeₓO_{y}, CoFeₓO_{y}, NiFeₓO_{y}, CuFeₓO_{y}, ZnFeₓO_{y},, CdFeₓO_{y}, BaFeₓO and SrFeₓO, wherein x and y vary depending on the method of synthesis, and wherein x is preferably an integer of from 1 to 3, more preferably 2, and wherein y is preferably 3 or 4, and most preferably, the at least one magnetic core (M) comprises Fe₃O₄.

Thus, the present invention also relates to a method, as described above, and a magnetic particle obtained or obtainable by said method, wherein the at least one magnetic core (M comprises a metal oxide or a metal carbide, more preferably, the at least one magnetic core (M) comprises an iron oxide, in particular an iron oxide selected from the group consisting of Fe₃O₄, α-Fe₂O₃, γ- Fe₂O₃, MnFeₓO_{y}, CoFeₓO_{y}, NiFeₓO_{y}, CuFeₓO_{y}, ZnFeₓO_{y},, CdFeₓO_{y}, BaFeₓO and SrFeₓO, wherein x and y vary depending on the method of synthesis, and wherein x is preferably an integer of from 1 to 3, more preferably 2, and wherein y is preferably 3 or 4, and most preferably, the at least one magnetic core (M) comprises Fe₃O₄

As described above, the magnetic core (M) preferably comprises, more preferably consists of, magnetic nanoparticles and a coating C1.

Preferably, step (i) comprises:
(i.1) providing at least one magnetic nanoparticle, and
(i.2) coating the at least one magnetic nanoparticle with a coating C1, the coating C1 preferably being selected from a group consisting of tensides, silica, silicates, silanes, phosphates, phosphonates, phosphonic acids and mixtures of two or more thereof to give the magnetic core (M).

The magnetic nanoparticle in (i.1), comprises, preferably consists of, at least one magnetic, preferably superparamagnetic, nanoparticle and optionally a coating C2.

Thus, step (i.1) comprises the provision of at least one nanoparticle.

Methods to provide magnetic nanoparticles are known to the skilled person and are e.g. described in Lu, Salabas, Schüth, Angew. Chem Int. Ed. 2007, 46, 1222-1244.

In particular in case of Fe₃O₄ nanoparticles, Fe(II) and Fe(III), oxide is precipitated from an aqueous, preferably from alkaline aqueous, media to give the respective nanoparticle.

According to the first preferred embodiment, described above, wherein each magnetic core (M) comprises at least one magnetic nanoparticle, optionally with a coating C1, step (i. 1) thus comprises the provision of at least one magnetic nanoparticle, optionally with a coating C1, forming the magnetic core (M). Preferably, in this case, the magnetic nanoparticle does not comprise a coating C2. According to this first preferred embodiment of the invention, the magnetic particles according to the invention comprise preferably of from 1 to 20 magnetic nanoparticles.

According to this embodiment, it is preferred that in case more than one magnetic cores (M) are present in the magnetic particle, these magnetic cores (M) are not aggregated with each other. Preferably, these particles are substantially evenly distributed within the polymer matrix.

Thus, the present invention also relates to a method, as described above, as well as to a magnetic particle, as described above, wherein step (i) comprises:
(i.1) providing at least one magnetic nanoparticle optionally with a coating C2, to give the at least one magnetic nanoparticle, and
(i.2) coating the at least one magnetic nanoparticle with a coating C1, the coating C1 preferably being selected from the group consisting of tensides, silica, silicates, silanes, phosphates, phosphonates, phosphonic acids and mixtures of two or more thereof to give the magnetic core (M).

According to the second preferred embodiment described above, the magnetic core (M), i.e. preferably each magnetic core (M), comprises more than 20 nanoparticles, preferably, between 100 to 1.5 million nanoparticles more preferably 750 -750,000 nanoparticles, more preferably 1,750 - 320,000 nanoparticles, in particular 90,000 - 320,000 nanoparticles. More preferably, the nanoparticles form at least one supraparticle consisting of aggregated, coated, nanoparticles. Preferably, said supraparticle being coated with at least one coating C1. Preferably in this case, the supraparticle is coated with at least one coating C1, wherein the coating is preferably deposited on the surface of the supraparticle. Thus, the supraparticle is preferably coated with a coating C1. It is to be understood that each nanoparticle within the supraparticle is preferably coated, such as with a coating C2. Step (i. 1) thus comprises the provision of more than 20 nanoparticles, wherein the method further comprises aggregating said nanoparticles thereby forming at least one supraparticle and optionally coating each supraparticle with at least one coating C1.

Thus, the present invention also relates to a method, as described above, as well as to a magnetic particle, as described above, wherein step (i) comprises:
(i.1) providing at least one supraparticle by
   (i.1.1) providing more than 20 nanoparticles, preferably with a coating C2,
   (i.1.2) aggregating said nanoparticles, thereby forming a supraparticle, and
(i.2) coating the at least one supraparticle with a coating C1, the coating C1 preferably being selected from the group consisting of tensides, silica, silicates, silanes, phosphates, phosphonates, phosphonic acids and mixtures of two or more thereof
to give the magnetic core (M).

In this case, each magnetic core (M) comprises a supraparticle preferably consisting of at least 21, coated, nanoparticles, preferably of 100 to 1.5 million nanoparticles more preferably of 750 -750,000 nanoparticles, more preferably of 1,750 - 320,000 nanoparticles, in particular of 90,000 - 320,000, coated, nanoparticles being aggregated with each other.

It is to be understood that (i.1.1) and (i. 1.2) may be carried out in a single step that is via a reaction in which supraparticles are directly provided.

Methods to provide supraparticles are known to the skilled person and are e.g. described in Liu et al., Angew. Chem. Int. Ed. 2009, 48, 5875 -5879.

In particular in case of supraparticles comprising Fe₃O₄ nanoparticles the synthesis may comprise the partial reduction of FeCl₃, preferably at elevated temperatures, such as at a temperature in the range of from 150 °C to 250 °C, and preferably at an increased pressure, such as a pressure of 2-10 bar, to obtain Fe₃O₄-supraparticles.

As described above, the coating C2 is thereby selected from at least one member of the group consisting of dicarboxylic acids, dicarboxylic acid salts, dicarboxylic acid derivatives, polyacrylic acid, polyacrylic acid salts, polyacrylic acid derivatives, tricarboxylic acids, tricarboxylic acid salts, tricarboxylic derivatives, amino acids, amino acid salts, amino acid derivatives, surfactants, salt of surfactants, fatty acids, fatty acid salts and fatty acid derivatives.

Methods to coat nanoparticles or supraparticles are known to the skilled person. The coating is preferably carried in situ during the synthesis of the supraparticle.

### Step (ii)

In step (ii) polymer precursor molecules are provided, i.e. monomeric building blocks which after polymerization give the respective polymer are provided.

Preferably, these polymer precursor molecules in (ii) are selected from the group consiting of styrene, functionalized styrenes, vinylbenzylchloride, divinylbenzene, vinylacetate, methylmethaacrylate and acrylic acid, more preferably selected from the group consisting of the following monomers: 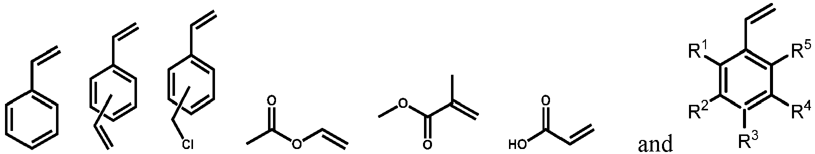 with R¹, R², R³, R⁴ and R⁵, are, independently of each other selected from the group consisting of -N₃, -NH₂, -Br, -I, -F, -NR'R'', -NR'R''R''', -COOH, -CN, -OH, -OR', -COOR', - NO₂, -SH2, -SO₂, -R'(OH)ₓ, -R'(COOH)ₓ, -R'(COOR'')ₓ, -R'(OR'')x, -R'(NH₂)ₓ, - R'(NHR")ₓ, -R'(NR"R‴)ₓ, -R'(Cl)ₓ, -R'(I)ₓ, -R'(Br)ₓ, -R'(F)ₓ, R'(CN)ₓ, -R'(N₃)ₓ, - R'(NO₂)ₓ, -R'(SH₂)ₓ, -R'(SO₂)ₓ, alkyl, aryl, cycloalkyl, heteroaryl, heterocycloalkyl and with R', Rʺ and R‴ being, independently of each other, selected from the group consisting of alkyl, aryl, cycloalkyl, heteroaryl, heterocycloalkyl, halides, hydrogen, sulfides, nitrates and amines.

### Step (iii)

In step (iii), the polymer precursor molecules according to (ii) are polymerized in the presence of the at least one magnetic core (M), thereby forming a particle comprising the at least one magnetic core (M), preferably the at least two magnetic cores (M), embedded in a polymer matrix (P1), wherein the polymer matrix (P1) preferably comprises, more preferably consists of, a crosslinked polymer, as described above and below. This crosslinked polymer matrix P1 is then hypercrosslinked in step (iv) to give the polymer matrix P.

The polymerization in (iii) is preferably a suspension polymerization. The term "suspension polymerization" refers to a system in which polymeric precursor molecules that are relatively insoluble in water are suspended as liquid droplets in an aqueous phase. Usually, a suspending agent is employed so as to maintain the suspension, and the resultant polymer is obtained as a dispersed solid phase. While the monomeric building blocks may be directly dispersed in a suspension polymerization system, hydrocarbon solvents or diluents are commonly employed with the monomers, such as n-heptane, isooctane, cyclohexane, benzene, toluene, and the like, including mixtures.

In the suspension polymerization system, a monomer mixture to be polymerized usually comprises the monomers, or, where desired, a polymer-in-monomer solution, the at least one magnetic core (M), solvent and, where employed, an initiator.

Thus, step (iii) preferably comprises:
(iii.1) providing a composition (A) comprising the polymer precursor molecules according to (ii), the at least one magnetic core (M) according to (i), at least one organic solvent, at least one initiator and a water phase and optionally additives, wherein the organic solvent is not miscible with water, and
(iii.2) stirring composition (A) to give an emulsion (B) wherein the emulsion is preferably an organic solvent-in-water emulsion.

Preferably, the polymerization in (iii) is carried out in the presence of an initiator selected from the group consisting of azobis(isobutyronitril) (AIBN), 2,2'-azodi(2-methylbutyronitrile) (VAZO 67), 1,1'-azobis(cyanocyclohexane) (VAZO 88), benzoylperoxid (BPO), 2,2'-azobis(2-amidinopropane) dihydrochloride (AAPH) and 4,4'-azobis(4-cyanopentanoic acid) (ACVA).

The monomers and the at least one magnetic core (M) are preferably suspended in a water solution optionally containing at least one suspending agent. The amount of water employed can vary widely, depending on the type of reactor employed, agitation means, and the like, though the final suspension mixture preferably contains about 5 to 60 per cent by weight of the monomeric building blocks based on total weight of the entire mixture including water.

A variety of suspending agents can be employed as additives in suspension polymerization systems, since the method involves a liquid-in-liquid dispersion and affords a final product in the form of discrete solid particles. The suspension agents include insoluble carbonates, silicates, talc, gelatine, pectin, starch, cellulose derivatives, insoluble phosphates, PVA, salts, NaCl, KCl, PVP and the like. Preferably, the polymerization in (iii) is carried out in the absence of any tensides.

The time employed for polymerization should be that sufficient for the degree or extent of conversion desired, and can vary over a wide range, depending on various reaction parameters such as the temperature employed, from a very few minutes to many hours, such as 48 hours. Preferably, step (iii) is carried out for a time in the range of from 1 hour to 30 hours, preferably 1 hour to 8 hours.

Temperatures employed are at least sufficient to effectuate thermal polymerization, or to cause decomposition of the free radical initiator, where used, which provides initiation of the reaction, preferably below temperatures which might cause gel formation of the polymer.

Temperatures preferably employed are in the range of about 0°C to 100°C, preferably 40 to 90°C.

The stirring is preferably carried out with an overhead stirrer.

Preferably, the polymer matrix (P1) comprises a crosslinked polymer, this polymer more preferably being obtained or obtainable by co-polymerizing a polymer with crosslinking agent. Suitable agents for crosslinking polymers are known to those skilled in the art, and include, but are not limited to divinylbenzene, bis(vinylphenyl)ethane, bis(vinylben-zyloxy)hexane, bis(vinylbenzyloxy)dodecane and derivatives of those.

Divinylbenzene is employed as crosslinking agent. The polymer matrix (P1) is obtained or obtainable by crosslinking a polymer with 5-90 vol% of a divinyl benzene crosslinking agent, based on the total amount of the polymer, wherein in the resulting polymer a cross-linking degree of at least 5% is obtained.

### Step (iv)

The polymer matrix (P1) is hypercrosslinked in a further step (iv). However, according to a particularly preferred embodiment, a hypercrosslinking in (iv) is carried out, preferably via a Friedel-Crafts reaction. The term "Friedel-Crafts reaction" for the purposes of this application, refers to a well-known reaction type developed by Charles Friedel and James Crafts to attach substituents to an aromatic ring by electrophilic aromatic substitution and includes the two main types of Friedel-Crafts reactions: alkylation reactions and acylation reactions. Alkylation may be preferably used in the invention. Such reactions are usually carried out in the presence of a suitable catalyst, such as a Lewis acid.

Preferably, (iv) is carried out in the presence of a catalyst comprising, more preferably consisting of, a Lewis acid selected from the group consisting of FeCl₃, ZnCl₂, AlCl₃, BF₃, SbCl₅, SnCl₄, TiCl₄, SiCl₄ and mixtures of two or more thereof. More preferably, the catalyst comprises, more preferably consists of, FeCl₃ or ZnCl₂ or a mixture thereof.

The hypercrosslinking in (iv) is carried out at a temperature in the range of from -30°C to 120°C, more preferably the hypercrosslinking in (iv) is carried out at a temperature of less than or equal to 80°C, such as a temperature in the range of from -30°C to 80°C.

More preferably (iv) is carried out a temperature of less than 70°C, more preferably at less than 60°C, more preferably at less than 50°C, more preferably at less than 40°C, more preferably at less than 30°C, such as preferably at a temperature in the range of from -30°C to 30°C, more preferably at a temperature in the range of from -20°C to 30°C, more preferably at a temperature in the range of from -20°C to 30°C, more preferably at a temperature in the range of from -10°C to 30°C, more preferably at a temperature in the range of from 0°C to 30°C and most preferably in the range of from 10 to 30°C. During the reaction, the temperature may be varied, constantly or stepwise, or held essentially constant. In case the temperature is varied, it is to be understood that the temperature is always kept at a temperature being equal to or less than 120°C, less than 100°C, less than 80°C, and preferably at a temperature of less than 70°C, more preferably at less than 60°C, more preferably at less than 50°C, more preferably at less than 40°C, more preferably at less than 30°C, such as preferably at a temperature in the range of from -30°C to 30°C, more preferably at a temperature in the range of from -20°C to 30°C, more preferably at a temperature in the range of from - 20°C to 30°C, more preferably at a temperature in the range of from -10°C to 30°C, more preferably at a temperature in the range of from 0°C to 30°C and most preferably in the range of from 10 to 30°C.

The reaction in (iv) is not carried in a solvent comprising dichloroethane or other organic halides.

The reaction in (iv) is carried out in a solvent comprising at least THF, acetonitrile, DMF, dioxane or toluol. Preferably, (iv) is carried out in a solvent selected from the group consisting of THF, acetonitrile, DMF, dioxane, toluol, preferably mixtures of two or more thereof.

Preferably, the reaction in (iv) is carried for a reaction time of 4h or less, such as for a time in the range of from 10 min to 2 h, more preferably 30 min to 1.5 h, more preferably 45 min to 1 h.

Preferably, the reaction in (iv) is carried out under inert atmosphere, even more preferably during the reaction in (iv) an inert gas is streamed through the mixture. Preferably, the inert gas is nitrogen and/or argon.

### Optional step (v)

Besides the steps described above, the method may comprise one or more further steps. In particular, the method may comprise functionalizing the surface of the polymeric particles obtained according to (iii) or (iv).

Thus, the present invention also relates to a method, as described above, as well as to magnetic particles obtained or obtainable by the above-described method, wherein the method further comprises:
(v) functionalizing the surface of the polymer particle according to (iii) or (iv).

Preferably in this step, the polymer particles are functionalized with a group selected from the group consisting of -OH, -COOH, diethylaminoethanol, R-SO₂-OH, -NH₂, R-SO₂-OH, - RNH, -R₂N, -R₃N⁺ -CH₃, -C₂H₅, -C₄H₉, -C₈H₁₇, -C₁₈H₃₇, -C₆H₅, -C₆H₉NO₆, Phenyl-Hexyl, Bi-Phenyl, Hydroxyapatit, boronic acid, biotin, azide, epoxide, alkyl, aryl, cycloalkyl, heteroaryl, heterocycloalkyl, aminoacids, -COOR, -COR, -OR, antibodies and fragments thereof, aptameres, nucleic acids, polymers and receptor proteins or binding domains thereof.

Methods for functionalizing polymeric particles are known to the skilled person and are e.g. described in Bioconjugate Techniques 2^{nd} Edition, G. T. Hermanson.

Preferably this step comprises at least the treatment of the particles with a suitable base (OH functionalization), such as e.g. KOH. Alternatively, functionalization can be a COOH-functionalization, a Cₓ-functionalization or an Epoxy-functionalization. Further details on said functionalizations are also given in the accompanying examples, below.

### Use/ Method of analyzing

The magnetic particle described above and the magnetic particles obtainable or obtainable by the method described above are preferably used for qualitative and/or quantitative determination of at least one analyte in a fluid.

The term "qualitative" determination as used herein refers to determining the presence or absence of at least one analyte in the fluid. Moreover, the term may also encompass an assessment of the nature of the analyte, i.e. it may encompass the identification of the analyte or the identification of a class of chemical molecules to which the analyte belongs.

The presence or absence of the at least one analyte can be determined by contacting the fluid sample to the magnetic particles for a time and under conditions sufficient to allow for binding of the at least one analyte to the magnetic particle, subsequently removing the remaining fluid sample from the magnetic particle and determining whether the at least one analyte was bound to the magnetic particle, or not. In order to determine whether the analyte was bound to the magnetic particle, or not, compounds bound to the particle may be eluted by suitable techniques and the presence or absence of the at least one analyte may be subsequently determined in the eluate. Alternatively, the binding at least one analyte may be determined directly, i.e. bound to the magnetic particle.

The identification of the at least one analyte or the chemical class to which it belongs may be done after the said analyte has been eluted from the magnetic particle by suitable analytical methods such as mass spectrometry, UV-vis, NMR, IR or biochemical methods, such as ELISA, RIA and the like. Alternatively, depending on the kind of agent used for the surface functionalization, only a specific analyte may be bound. For example, in the case of antibodies, the chemical nature of the analyte is predetermined due to the antibody's specificity.

The term "quantitative" as used herein refers to determining the absolute or relative amount of the at least one analyte comprised in a fluid sample.

The amount of the at least one analyte can be determined as described above for the qualitative determination. However, after elution of the analyte from the magnetic particles, the amount is to be determined in the eluate. Alternatively, the amount of bound analyte may be determined directly.

In light of the above, the present invention also contemplates a method for determining at least one analyte in a fluid sample comprising the steps of:
(a) contacting a magnetic particle according to the invention or the magnetic particle obtained or obtainable by the method of the present invention with a fluid sample comprising or suspected to comprise the at least one analyte; and
(b) determining the at least one analyte eluted from the said magnetic particle.

Typically, the determination referred to in this context is a qualitative or quantitative determination.

Typically, step (a) of the method is carried out for a time and under conditions sufficient to allow for binding of the at least one analyte to the magnetic particle. Thus, preferably in step (a) at least a portion, preferably all of, the analyte is bounded to the particle. In case, the determination is a quantitative determination, preferably substantially all of the anlalyte present in the fluid sample is bound to the particle.

Preferably, step (a) further comprises step:
(a1) washing the magnetic particle to which at least a portion of the at least one analyte is bounded to, preferably under conditions which do not elute the at least one analyte; and/or
(a2) eluting the at least one analyte from the magnetic particle under conditions suitable to allow the elution of the at least one analyte.

More specifically, the qualitative or quantitative determination in (b) may comprise the determination of the presence or absence of bound analyte on the magnetic particle or the determination of the amount of analyte bound to the magnetic particle.

It is to be understood that the washing step in (a1) may be carried as single washing step. Alternatively more than one washing step may be carried out.

More specifically, the qualitative determination may comprise the following further step as part of step (a) and/or (b):
- determining whether the at least one analyte was bound to the magnetic particle, or not.

Using the magnetic particles of the invention or obtainable by the method of the invention, advantageously reduces the matrix carry-over of the said fluids in applications as discussed above.

Analytes to be determined by the magnetic particles of the invention or the magnetic particles obtained by the method of the invention or analytes to be determined in accordance with the aforementioned uses are, preferably, chemical compounds present in biological fluid samples, environmental samples or solutions of mixtures of chemical compounds. Accordingly, the fluid sample referred to in accordance with the present invention is, preferably, selected from the group of fluids consisting of: body fluids, liquid or dissolved environmental samples and solutions of mixtures of chemical compounds.

In a preferred embodiment the fluid sample as used herein refers to a biological sample obtained for the purpose of evaluation in vitro. In the methods of the present invention, the fluid sample or patient sample preferably may comprise any body fluid.

Preferred fluid samples are whole blood, serum, plasma, bronchioalveolar lavage (BAL), epithelial lining fluid (ELF), urine or sputum, with plasma or serum being most preferred.

The term fluid sample includes biological samples that have been manipulated in any way after their procurement, such as by treatment with reagents, solubilization, or enrichment for certain components, such as proteins or polynucleotides. Typically, the fluid sample is a liquid sample.

The fluid sample may for example be whole blood, serum, antibodies recovered from the patient or plasma. The fluid sample is preferably whole blood, serum or plasma. In one embodiment, the sample is a clinical sample. In another embodiment, the sample is used in a diagnostic assay.

Depending on the nature of the fluid sample, different classes of chemical compounds are to be detected. Preferably, the analyte in accordance with the present invention may be selected from the group of steroids, sugars, vitamins, drugs including medicaments and drugs for abuse, organic compounds, proteins, nucleic acids and mixtures of two or more thereof. Such analytes, pivotally, occur in biological samples. However, they may also be present in environmental samples.

The aforementioned applications for determining analytes in fluid samples may, preferably, be applied or are involved in diagnostic purposes, drug of abuse testing, environmental control, food safety, quality control, purification or manufacturing processes. In diagnostic applications, the qualitative or quantitative determination of an analyte may allow aiding the diagnosis if the analyte is, e.g., a biomarker for a disease or medical condition. Similarly, the qualitative or quantitative assessment of an analyte being an indicator for environmental changes may help to identify pollution or to make assessments of environmental changes. Food safety as well as manufacturing or purification processes may be controlled by qualitative or quantitative determination of indicator analytes. Such indicators may also be determined in connection with general aspects of quality control, e.g., also in storage stability assessments of products and the like.

Preferably, the analyte is determined by mass spectrometry, UV-vis, NMR, IR.

Summarizing the findings of the present invention, the following embodiments are particularly preferred:
Embodiment (1): Magnetic particle comprising a polymer matrix (P) and at least one magnetic core (M), preferably at least two magnetic cores, wherein the polymer matrix comprises a hypercrosslinked polymer and wherein the magnetic particle has a particle size in the range of from 5 to 40 micrometers, as determined according to ISO 13320;
   wherein the polymer matrix (P) comprises a co-polymer obtained or obtainable by a method comprising co-polymerizing suitable monomeric building blocks in the presence of one monomeric building block which is a crosslinking agent, wherein 5-90 vol% of all monomeric building blocks are divinylbenzenes;
   wherein in the co-polymerization a polymer with a crosslinking degree of at least 5% is obtained;
   wherein divinylbenzene is the crosslinking agentwherein the suitable monomeric building blocks are selected from the group consisting of styrene, functionalized styrenes, vinylbenzylchloride, divinylbenzene, vinylacetate, methylmethaacrylate and acrylic acid;
   wherein the at least one magnetic core (M) comprises at least one iron oxide nanoparticle;
   wherein the at least one magnetic core (M) comprises at least one magnetic nanoparticle and a coating C1;
   wherein the coating C1 is tenside or silica coating.
Embodiment (2): Magnetic particle of embodiment (1), wherein the polymer matrix comprises pores having a pore size smaller than 100 nm, preferably smaller or equal to 50 nm, as determined according to ISO 15901-3.
Embodiment (3): Magnetic particle of embodiment (2), wherein at least 90% of all pores present in the polymer matrix (P) have a pore size smaller than 10 nm and at least 50% of all pores present in the polymer matrix have a pore size smaller than 5 nm, as determined according to ISO 15901-3, preferably, wherein the polymer matrix (P) does not comprise macropores having a pore size larger than 50 nm.
Embodiment (4): Magnetic particle of embodiment (2) or (3), wherein the particle has a BET specific surface area in the range of from 50 to 2500 m²/g, as determined according to ISO 9277.
Embodiment (5): Magnetic particle of any one of embodiments (1) to (4), wherein the magnetic particle has a saturation magnetization of at least 1 A m²/kg, preferably of at least 10 A m²/kg, as determined according to ASTM A 894/A 894M.
Embodiment (6): Magnetic particle of any one of embodiments (1) to (5), wherein the at least one magnetic core (M) comprises a compound selected from the group consisting of metal carbide, metal nitride, metal sulfide, metal phosphide, metal chelate and a mixture of two or more thereof.
Embodiment (7): Magnetic particle of any one of embodiments (1) to (6), wherein the at least one magnetic core (M) comprises an iron oxide selected from the group consisting of Fe₃O₄, α-Fe₂O₃, γ- Fe₂O₃, MnFeₓO_{y}, CoFeₓO_{y}, NiFeₓO_{y}, CuFeₓO_{y}, ZnFeₓO_{y},, CdFeₓO_{y}, BaFeₓO and SrFeₓO, wherein x and y vary depending on the method of synthesis, and wherein x is preferably an integer of from 1 to 3, more preferably 2, and wherein y is preferably 3 or 4 most preferably, Fe₃O₄.
Embodiment (8): Magnetic particle of any one of embodiments (1) to (7), wherein the magnetic particle is superparamagnetic.
Embodiment (9): Magnetic particle of any one of embodiments (1) to (8), wherein the at least one magnetic core (M) comprises a Fe₃O₄-nanoparticle.
Embodiment 10: Magnetic particle of embodiment (9), wherein the at least one magnetic nanoparticle has a diameter in the range of from 1 to 20 nm, preferably 4 to 15 nm, as determined according to TEM-measurements.
Embodiment (11): Magnetic particle of any one of embodiments (1) to (8), wherein the at least one magnetic core (M) comprises, preferably consists of, a supraparticle comprising a coating C1.
Embodiment (12): Magnetic particle of embodiment (11), wherein said supraparticle consists of aggregated nanoparticles and, preferably, of more than 20 aggregated nanoparticles and, more preferably, of between 100 and 1.5 million nanoparticles.
Embodiment (13): The magnetic particle of embodiment (12), wherein each nanoparticle is coated with at least one coating C2, which coating preferably is deposited on the surface of the nanoparticle,
   and wherein the coating C2 is preferably selected from the group consisting of dicarboxylic acids, dicarboxylic acid salts, dicarboxylic acid derivatives, tricarboxylic acids, tricarboxylic acid salts, tricarboxylic derivatives, amino acids, amino acid salts, amino acid derivatives, surfactants, salt of surfactants, polyacrylic acid, polyacrylic acid salts, polyacrylic acid derivatives, fatty acids, fatty acid salts and fatty acid derivatives.
Embodiment (14): Magnetic particle of any one of embodiments (11) to (13), wherein the supraparticle including the at least one coating C2, has a diameter in the range of from 80 to 500 nm, preferably 150 to 400 nm, and most preferably 200 to 300 nm, as determined according to DLS.
Embodiment (15): Magnetic particle of any one of embodiments (10) to (14), wherein the at least one magnetic core (M) comprises the coating C1 in an amount of from 1 to 40% by weight, preferably from 2 to 15% by weight, more preferably from 5 to 10% by weight, based on the total weight of at least one magnetic core (M).
Embodiment (16): Magnetic particle of any one of embodiments (10) to (11), wherein the coating C1 is a tenside, and wherein the at least one magnetic core (M) preferably comprises the coating C1 in an amount of from 1 to 15% by weight, preferably from 5 to 10% by weight, based on the total weight of at least one magnetic core (M), preferably an oleic acid coating.
Embodiment (17): Magnetic particle of any one of embodiments (1) to (16), wherein the particle comprises at least two magnetic cores (M).
Embodiment (18): Magnetic particle of any one of embodiments (1) to (17), wherein at least one magnetic core (M), preferably at least two magnetic cores (M), are embedded in the polymer matrix.
Embodiment (19): Magnetic particle of any one of embodiments (1) to (18), wherein the particle comprises at least two magnetic cores (M) and a polymer matrix (P).
Embodiment (20): Magnetic particle of ay one embodiments (1) to (19), wherein the surface of the particle is functionalized with at least one group selected from the group consisting of -OH, -COOH, diethylaminoethanol, R-SO₂-OH, -NH₂, R-SO₂-OH, -RNH, -R₂N, -R₃N⁺ - CH₃, -C₂H₅, -C₄H₉, -C₈H₁₇, -C₁₈H₃₇, -C₆H₅, C₆H₉NO₆ Phenyl-Hexyl, Bi-Phenyl, Hydroxyapatit, boronic acid, biotin, azide, epoxide, alkyl, aryl, cycloalkyl, heteroaryl, heterocycloalkyl, aminoacids, -COOR, -COR, -OR, antibodies and fragments thereof, aptameres, nucleic acids, and receptor proteins or binding domains thereof.
Embodiment (21): Magnetic particle of any one of embodiments (1) to (20), wherein the particle is a substantially spherical particle.
Embodiment (22): A method of preparing a magnetic particle comprising a polymer matrix (P) and at least one magnetic core (M), preferably at least two magnetic cores (M), wherein the polymer matrix (P) comprises at least one crosslinked polymer, wherein the magnetic particle has a particle size in the range of from 5 to 40 micrometers, as determined according to ISO 13320, the method comprising:
   (i) providing at least one magnetic core (M), preferably at least two magnetic cores (M),
   (ii) providing polymer precursor molecules,
   (iii) polymerizing the polymer precursor molecules according to (ii) in the presence of the at least one magnetic core (M), thereby forming a particle comprising the at least one magnetic core (M), preferably the at least two magnetic cores (M), embedded in a polymer matrix (P1), wherein the polymer matric (P1) comprises, more preferably consists of, a crosslinked polymer, and
   (iv) hypercrosslinking the polymer matrix (P1) of the polymer particle obtained in (iii), to give the magnetic particle;

   wherein the reaction in (iv) is not carried out in a solvent comprising dichloroethane or other organic halides;
   wherein in (iii) the polymer matrix (P1) comprises a crosslinked polymer being obtained or obtainable by crosslinking a polymer with 5-90 vol% of a divinylbenzene crosslinking agent, based on the total amount of the polymer, wherein in the resulting polymer a crosslinking degree of at least 5% is obtained;
   wherein divinylbenzene is the crosslinking agent;
   wherein the hypercrosslinking, in (iv) is carried out at a temperature in the range of from - 30 to 120°C;
   wherein the reaction in (iv) is carried out in a solvent comprising at least THF, acetonitrile, DMF, dioxane or toluol.
Embodiment (23): The method of embodiment (22), wherein in (iii), the at least magnetic core (M) is embedded into the matrix.
Embodiment (24): The method of any one of embodiments (22) or (23), wherein the hypercrosslinking, in (iv) is carried out via a Friedel-Crafts reaction.
Embodiment (25): The method of any one of embodiments (22) to (24), wherein the hypercrosslinking in (iv) is carried out in the presence of a catalyst comprising, more preferably consisting of, a Lewis acid selected from the group consisting, of FeCl₃, ZnCl₂, AlCl₃, BF₃, SbCl₅, SnCl₄, TiCl₄, SiCl₄ and mixtures of two or more thereof, more preferably in the presence of a catalyst comprising, more preferably consisting of, FeCl₃ or ZnCl₂ or a mixtures thereof.
Embodiment (26): The method of any one of embodiments (22) to (25), wherein the method further comprises:
   (v) functionalizing the surface of the polymer particle according to (iii) or (iv).
Embodiment (27): The method of embodiment (26), wherein in step (v), the polymer particle is functionalized with a group selected from the group consisting of -OH, -COOH, diethylaminoethanol, , R-SO₂-OH, -NH₂, R-SO₂-OH, -RNH, -R₂N, -R₃N⁺ -CH₃, -C₂H₅, -C₄H₉, - C₈H₁₇, -C₁₈H₃₇, -C₆H₅, C₆H₉NO₆ Phenyl-Hexyl, Bi-Phenyl, Hydroxyapatit, boronic acid, biotin, azide, epoxide, alkyl, aryl, cycloalkyl, heteroaryl, heterocycloalkyl, aminoacids, - COOR, -COR, -OR, antibodies and fragments thereof, aptameres, nucleic acids, and receptor proteins or binding domains thereof.
Embodiment (28): The method of any one of embodiments (22) to (27), wherein the polymerization in (iii) is a suspension polymerization.
Embodiment (29): The method of any one of embodiments (22) to (28), wherein the polymerization in (iii) is carried out in the presence of an initiator selected from the group consisting of azobis(isobutyronitril) (AlBN), 2,2'-azodi(2-methylbutyronitrile) (VAZO 67), 1,1'-azobis(cyanocyclohexane) (VAZO 88), benzoylperoxid (BPO), 2,2'-azobis(2-amidinopropane) dihydrochloride (AAPH) and 4,4'-azobis(4-cyanopentanoic acid) (ACVA).
Embodiment (30): The method of any one of embodiments (22) to (29), wherein step (iii) comprises:
   (iii.1) providing a composition (A) comprising the polymer precursor molecules according to (ii), the at least one magnetic core (M) according to (i), at least one organic solvent, at least one initiator and a water phase, wherein the organic solvent is not miscible with water, and wherein, optionally, the water comprises at least one additive; and
   (iii.2) stirring composition (A) to give an emulsion (B), wherein the emulsion is preferably an organic solvent-in-water emulsion.
Embodiment (31): The method of embodiment (30), wherein said at least one additive is selected from the group consisting of: insoluble carbonates, silicates, talc, gelatine, pectin, starch, cellulose derivatives, insoluble phosphates, PVA, salts, NaCl, KCl, and PVP.
Embodiment (32): The method of embodiment (30) or (31), wherein the stirring is carried out with an overhead stirrer.
Embodiment (33): The method of any one of embodiments (20) to (31), wherein the polymerization in (iii) is carried out in the absence of any tensides.
Embodiment (34): The method of any one of embodiments (22) to (33), wherein step (iii) is carried out for a time in the range of from 1 hour to 30 hours, preferably 1 hour to 8 hours.
Embodiment (35): The method of any one of embodiments (22) to (34), wherein step (iii) is carried out at a temperature in the range of from 0°C to 100°C, preferably 40°C to 90°C.
Embodiment (36): The method of any one of embodiments (22) to (35), wherein step (i) comprises:
   (i.1)providing at least one magnetic nanoparticle, and
   (i.2)coating the at least one magnetic nanoparticle with a coating C1, the coating C1 preferably being selected from the group consisting of tensides, silica, silicates, silanes, phosphates, phosphonates, phosphonic acids and mixtures of two or more thereof
   to give the at least one magnetic core (M).
Embodiment (37): The method of embodiment (36), wherein (i) comprises:
   (i.1.1) providing more than 20 nanoparticles and, preferably, at least 21 nanoparticles with at least one coating C2,
   (i.1.2) aggregating the more than 20 nanoparticles, thereby forming a supraparticle, and
   (i.1.3) optionally coating the supraparticle according to (i.2) with at least one coating C1, to give the at least one magnetic core (M).
Embodiment (38): The method of embodiment (37), wherein the at least one coating C2 is selected from the group consisting of dicarboxylic acids, dicarboxylic acid salts, dicarboxylic acid derivatives, polyacrylic acid, polyacrylic acid salts, polyacrylic acid derivatives, tricarboxylic acids, tricarboxylic acid salts, tricarboxylic derivatives, amino acids, amino acid salts, amino acid derivatives, surfactants, salt of surfactants, fatty acids, fatty acid salts and fatty acid derivatives.
Embodiment (39): Magnetic particle obtained or obtainable by a method of any one of embodiments (22) to (38), wherein the particle is preferably a substantially spherical particle.
Embodiment (40): Use of the magnetic particle of any one of embodiments (1) to (21) or a magnetic particle according to embodiment (39) for qualitative and/or quantitative determination of at least one analyte in a fluid.
Embodiment (41): The use of embodiment (40), wherein the said analyte is selected from the group of steroids, sugars, vitamins, drugs, organic compounds, proteins, nucleic acids, sugars and mixtures of two or more thereof.
Embodiment (42): The use of embodiment (40) or (41), wherein the said analyte is enriched by the magnetic particles of embodiments (1) to (21) or the magnetic particle according to embodiment (39).
Embodiment (43): The use of any one of embodiments (40) to (42), wherein the said analyte is determined by mass spectrometry, UV-vis, NMR, IR.
Embodiment (44): The use of any one of embodiments (40) to (43), wherein said fluid is selected from the group of fluids consisting of: body fluids, liquid or dissolved environmental samples and solutions of mixtures of chemical compounds.
Embodiment (45): The use of the preceding embodiment, wherein the magnetic particle of any one of embodiment (1) to (21) or the magnetic particle according to embodiment (39) reduces the matrix carry-over of the said fluids.
Embodiment (46): The use of embodiment (45), wherein said qualitative and/or quantitative determination of the at least one analyte in a fluid is involved in diagnostic purposes, drug of abuse testing, environmental control, food safety, quality control, purification or manufacturing processes.
Embodiment (47): A method for determining at least one analyte in a fluid sample comprising the steps of:
   a) contacting a magnetic particle of the invention or a magnetic particle obtained by the method of the present invention with a fluid sample comprising or suspected to comprise the at least one analyte; and
   b) determining the at least one analyte bound to the said magnetic particle.
Embodiment (48): The method of embodiment (47), wherein step a) of the method is carried out for a time and under conditions sufficient to allow for binding of the at least one analyte to the magnetic particle.
Embodiment (49): The method of embodiment (47) or (48), wherein step a) further comprises:
   a1) washing the magnetic particle to which at least a portion of the at least one analyte is bounded to, preferably under conditions which do not elute the at least one analyte; and/or
   a2) eluting the at least one analyte from the magnetic particle under conditions suitable to allow the elution of the at least one analyte.
Embodiment (50): The method of any one of embodiments (47) to (49), wherein said determination comprises the determination of the presence or absence of bound analyte on the magnetic particle or the determination of the amount of analyte bound to the magnetic particle.

The scope of the invention is not restricted by the preferred embodiments. The embodiments are schematically depicted in the Figures. Therein, identical reference numbers in these Figures refer to identical or functionally comparable elements.

### FIGURES

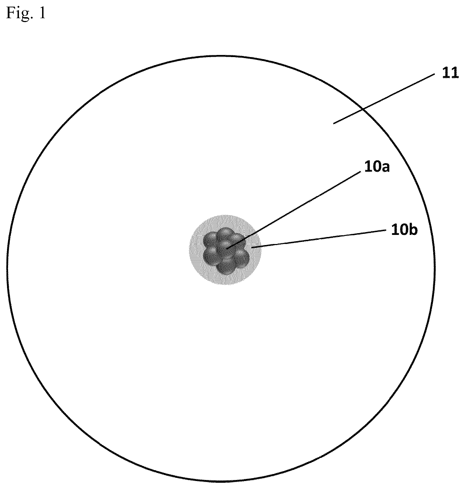

**Figure 1** is a schematic view of a magnetic particle comprising one magnetic core (M) (10a + 10b) with 1-20 nanoparticles (10a), and a coating C1 (10b) as well as a polymer matrix (P) (11)

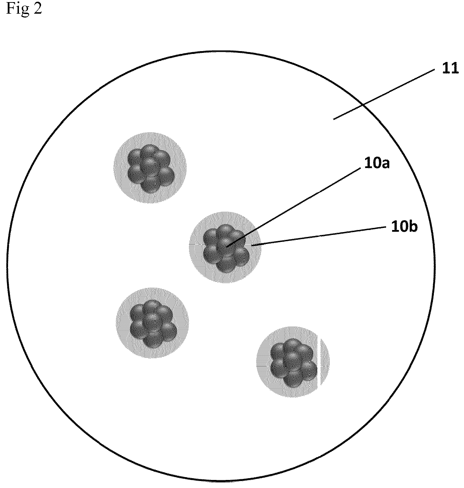

**Figure 2** is a schematic view of a more preferred magnetic particle comprising more than one, i.e. three magnetic cores (M) (10a + 10b), each with 1-20 nanoparticles (10a) and a coating C1 (10b) as well as a polymer matrix (P) (11)

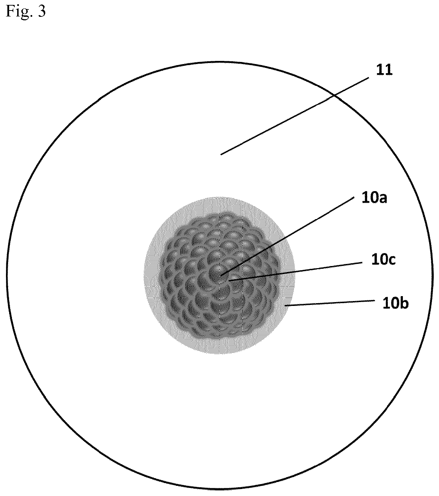

**Figure 3** is a schematic view of a magnetic particle with a polymer matrix (P) (11) and one magnetic core (M) consisting of a supraparticle formed upon aggregating of multiple nanoparticles (10a) which are coated with a coating C2 (10c), the magnetic core (M) further having a coating C1 (10b)

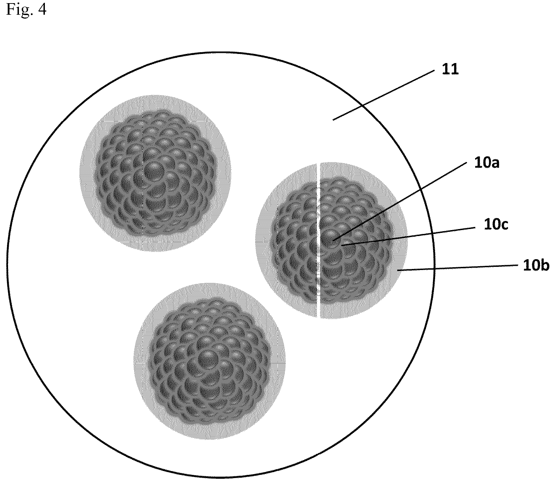

**Figure 4** is a schematic view of a more preferred magnetic particle with a polymer matrix (P) (11) and more than one magnetic core (M) consisting of a supraparticle formed upon aggregating of multiple nanoparticles (10a) which are coated with a coating C2 (10c), the magnetic core (M) further having a coating C1 (10b)
**Figure 5** (not according to the invention) shows the calculated saturation magnetization for the particles synthesized by **Xu *et al.*** with different sizes.

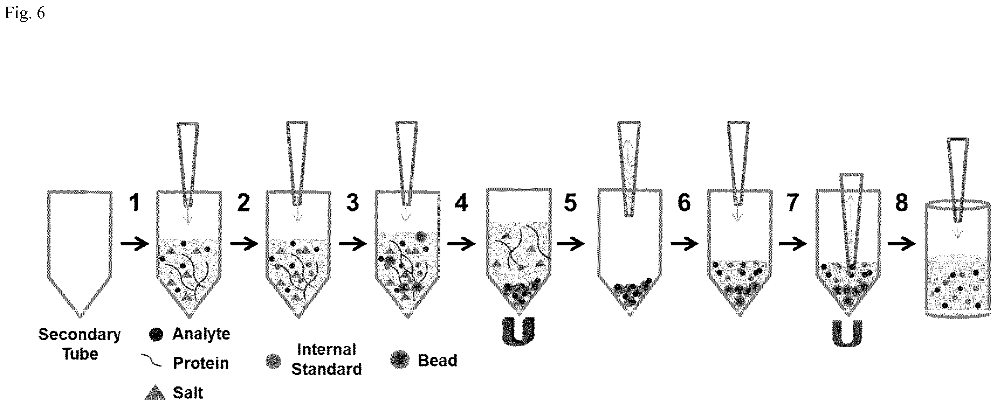

**Figure 6** shows an enrichment workflow using beads as described herein for sample analysis.

### EXAMPLES

The following Examples shall merely illustrate the invention. Whatsoever, they shall not be construed as limiting the scope of the invention, that is set out in the appended set of claims.

### Example 1: Preparation of beads

### Silica-coated magnetic nanoparticles (1)

In a general procedure 43 g FeCl₂ · 4 H₂O (0.22 mol) and 70 g FeCl₃ (0.43 mol) were added under stirring to 2 L water and heated to 70 °C. 125 mL NH₄OH (28% in H₂O) were added and after 10 min the black precipitate was separated with a magnet. The supernatant was discarded and the magnetic nanoparticles were washed five times with water. The nanoparticles were resuspended in 2 L ethanol under ultrasonication and transferred in a 4 L reactor. The total volume was filled to 3.2 L with ethanol and under stirring a mixture of tetraethoxysilane (TEOS; 50 mL; 0.22 mol) and ethanol (350 mL) was added drop wise. After 24 h stirring at 25 °C the nanoparticles were separated with a magnet and the supernatant was discarded. The TEOS coated nanoparticles were washed three times with ethanol and resuspended in 2 L ethanol under ultrasonication. The nanoparticles were transferred in a 4 L reactor and the total volume was filled to 3.1 L with ethanol. The mixture was heated to 50 °C and under stirring a mixture of [3-(methacryloyloxy)propyl]trimethoxysilane (MEMO; 77 mL; 0.32 mol), isobutyl(trimethoxy)silane (ISO; 33 mL; 0.17 mol) and ethanol (390 mL) was added drop wise. After 24 h stirring at 50 °C the nanoparticles were separated with a magnet and the supernatant was discarded. The MEMO/ISO coated nanoparticles were washed two times with ethanol and stored in ethanol to give *silica-coated magnetic nanoparticles* **(1)** (50 g).

### Tenside-coated magnetic nanoparticles (2)

In a general procedure 126 g FeCl₂ · 4 H₂O (0.63 mol) and 248 g FeCl₃ (1.53 mol) were added under stirring to 3 L water and heated to 55 °C. 460 mL NH₄OH (28% in H₂O) were added and after 15 min the black precipitate was separated with a magnet. The supernatant was discarded and the magnetic nanoparticles were washed three times with water. The magnetic nanoparticles were resuspended in 2000 mL and the pH was adjusted to 7-9 with NaOH (10 M). After ultrasonication for 30 min the suspension was transferred in a 4 L reactor and 1 L water was added. While stirring 120 mL oleic acid were added and the suspension was stirred for 45 min at 25 °C. The magnetic nanoparticles were separated with a magnet and the supernatant was discarded. The tenside-coated nanoparticles were washed three times with water and ethanol and stored in ethanol to give *tenside-coated magnetic nanoparticles* **(2)** (203 g).

### Silica-coated magnetic supraparticles (3)

In a general procedure 44 g FeCl₃ · 6 H₂O (0.16 mol) were dissolved in 800 mL ethylene glycol and transferred to high pressure reactor. 9.7 g sodium citrate (0.037 mol) and 51.9 g sodium acetate (0.63 mol) were added and the high pressure reactor was sealed. The mixture was treated with stirring at 160 °C for 2 h and at 200 °C for 18 h. The formed nanoparticles were separated with a magnet and the supernatant was discarded. The magnetic nanoparticles were washed three times with ethanol and five times with water to give magnetic supraparticles (13.3 g). 10 g of the supraparticles were resuspended in 1500 mL ethanol in a 2 L reactor under ultrasonication and while stirring a mixture of tetraethoxysilane (TEOS; 15 mL; 68 mmol) and ethanol (50 mL) was added drop wise. After 20 h stirring at 25 °C the supraparticles were separated with a magnet and the supernatant was discarded. The TEOS coated supraparticles were washed two times with ethanol and resuspended in 1300 mL ethanol under ultrasonication. The mixture was heated to 65 °C and under stirring a mixture of [3-(methacryloyloxy)propyl]trimethoxysilane (MEMO; 14 mL; 59 mmol), isobutyl(tri-methoxy)silane (ISO; 6 mL; 31 mmol) and ethanol (180 mL) was added drop wise. After 16 h stirring at 50 °C the supraparticles were separated with a magnet and the supernatant was discarded. The MEMO/ISO coated supraparticles were washed three times with ethanol and stored in ethanol to give *silica-coated magnetic supraparticles* **(3)** (10 g).

### Magnetic polymer particles (4)

In a general procedure 650 mL water was added to a 2 L glass reactor with mechanical stirrer. 13.5 g PVA was added and stirred until complete solution. 10 g of **(1), (2)** or **(3)** were separated with a magnet and the supernatant was discarded. The magnetic nanoparticles were washed one time with toluene and then resuspended in 168 mL toluene. 23.6 mL divinylbenzene (0.17 mol) and 23.6 mL vinylbenzyl chloride (0.17 mol) were added and the mixture was ultrasonicated for 1 h. 3.84 g 2,2'-azobis(2-methylbutyronitrile) (20 mmol) were added and the mixture was transferred to the PVA-solution under rapid stirring. The mixture was heated to 80 °C and the reaction continued for 4 h. The formed magnetic polymer particles were separated with a magnet and the supernatant was discarded. The particles were washed three times with water and methanol and resuspended in isopropanol/water (10/90 v/v) to give *magnetic polymer particles* **(4)** (52.3 g).

### Porous magnetic polymer particles (5)

In a general procedure 5 g of **(4)** were separated with a magnet and the supernatant was discarded. The magnetic nanoparticles were washed three times with the hypercrosslinking solvent (dichloroethane, toluene, DMF, MeCN, dioxane or THF) and then resuspended in 60 mL in the chosen solvent. The suspension was stirred for 30 min and then heated to 80 °C. When the temperature was reached the catalyst (FeCl₃ or ZnCl₂; 12 mmol) was added and nitrogen was bubbled through the suspension. After 1 h the particles were separated with a magnet and the supernatant was discarded. The particles were washed five times with ethanol to give *porous magnetic polymer particles* **(5)** (4.8 g).

### OH-functionalized porous magnetic polymer particles (6)

In a general procedure 4.8 g of **(5)** were used as synthesized and suspended in 60 mL KOH-solution (6 M in H₂O). The suspension was stirred at 60 °C for 16 h. The particles were separated with a magnet and the supernatant was discarded. The particles were washed several times with water until pH 7 was reached to give *OH-functionalized porous magnetic polymer particles* **(6)** (4.8 g).

### COOH-functionalized porous magnetic polymer particles (7)

In a general procedure 1 g of **(6)** were used as synthesized and suspended in 20 mL NaClO-solution (10-15% available chlorine in H₂O). The suspension was stirred at 60 °C for 1.5 h. The particles were separated with a magnet and the supernatant was discarded. The particles were washed three times with water to give *COOH-functionalized porous magnetic polymer particles* **(7)** (1 g).

### Cₓ-functionalized porous magnetic polymer particles (8)

In a general procedure 1 g of **(7)** were used as synthesized and washed two times with MES-buffer (2-morpholin-4-ylethanesulfonic acid; 0.1 M; pH 5.0). The particles were resuspended in 40 mL MES-buffer (0.1 M; pH 5.0) and 6.6 mL EDC-solution (3-(ethyliminomethyleneamino)-N,N-dimethylpropan-1-amine; 10% w/w in H₂O) and 39.6 mL sulfo-NHS-solution (N-hydroxysulfosuccinimide; 0.03 M in H₂O) were added. The suspension was stirred at 11 °C for 35 min. The particles were separated with a magnet and the supernatant was discarded. The particles were washed two times with MES-buffer and 46 mL of a CₓH₂ₓ₊₁-NH₂-solution (x = 4, 6, 8 or 10; 1/6 v/v in ethanol) and 49.5 mL potassium phosphate-buffer (0.1 M; pH 7.5) were added. The suspension was stirred at 25 °C for 2.5 h. The particles were separated with a magnet and the supernatant was discarded. The particles were washed three times with ethanol and two times with potassium phosphate-buffer (0.02 M; pH 7.0) to give *Cₓ-functionalized porous magnetic polymer particles* **(8)** (1 g).

### Epoxy-functionalized porous magnetic polymer particles (9)

In a general procedure 1 g of **(6)** were used as synthesized and suspended in 10 mL DMF. 20 mL epichlorohydrin and 10 mL NaOH-solution (3.5 M in H₂O) were added. The Suspension was stirred at 40 °C for 22 h. The particles were separated with a magnet and the supernatant was discarded. The particles were washed several times with water to give *epoxy-functionalized porous magnetic polymer particles* **(9)** (1 g).

The following Particles were prepared:

**Table 1: Prepared particles**

| **CPS - Hyper-cross-linked Bead Type** | **embedded Fe3O4 Type** | **Magnet-ite** | **VBC** | **DVB** | **St** | **VAc** | **PFSt** | **Part. Size X50,3** | **Ms** | **SSA** |
|---|---|---|---|---|---|---|---|---|---|---|
| | | | % | % | % | % | % | µm | Am²/kg | m²/g |
| **01-03** | **NPs@silica** | 1 | 50 | 50 | 0 | 0 | 0 | 21.5 | 10.4 | 1068 |
| **01-05** | **SupraPs@silica** | 3 | 50 | 50 | 0 | 0 | 0 | 27.0 | 7.1 | 1058 |
| **01-10** | **NPs@tenside** | 2 | 50 | 50 | 0 | 0 | 0 | 17.4 | 7.6 | 1037 |
| **04-13** | **NPs@silica** | 2 | 75 | 25 | 0 | 0 | 0 | 20.0 | 9.6 | 1131 |
| **04-12** | **SupraPs@silica** | 3 | 75 | 25 | 0 | 0 | 0 | 27.0 | 1.9 | 1617 |
| **33-02** | **NPs@silica** | 1 | 25 | 75 | 0 | 0 | 0 | 32.0 | 3.8 | 950 |
| **33-05** | **NPs@tenside** | 2 | 25 | 75 | 0 | 0 | 0 | 22.4 | 6.4 | 995 |
| **44-01** | **NPs@silica** | 1 | 37.5 | 62.5 | 0 | 0 | 0 | 29.6 | 3.9 | 2001 |
| **50-01** | **NPs@silica** | 1 | 10 | 90 | 0 | 0 | 0 | 37.4 | 4.0 | 940 |
| **05-22** | **NPs@silica** | 1 | 20 | 30 | 50 | 0 | 0 | 33.2 | 9.4 | 627 |
| **05-32** | **SupraPs@silica** | 3 2 | 20 | 30 | 50 | 0 | 0 | 21.6 | 6.3 | tbd |
| **05-36** | **NPs@tenside** | | 20 | 30 | 50 | 0 | 0 | 32.8 | 23.4 | 476 |
| **39-01** | **NPs@silica** | 1 | 37.5 | 5 | 57.5 | 0 | 0 | 29.7 | 16.3 | 58 |
| **40-01** | **NPs@silica** | 1 | 25 | 40 | 35 | 0 | 0 | 27.9 | 3.5 | 618 |
| **42-01** | **NPs@silica** | 1 | 50 | 27.5 | 22.5 | 0 | 0 | 24.5 | 5.3 | 1247 |
| **43-01** | **NPs@silica** | 1 | 50 | 5 | 45 | 0 | 0 | 19.2 | 4.8 | 817 |
| **46-01** | **NPs@silica** | 1 | 25 | 5 | 70 | 0 | 0 | 29.8 | 10.1 | 18 |
| **48-01** | **NPs@silica** | 1 | 10 | 65 | 25 | 0 | 0 | 40.1 | 5.0 | 924 |
| **49-01** | **NPs@silica** | 1 | 37.5 | 37.5 | 25 | 0 | 0 | 35.7 | 2.3 | 1201 |
| **51-01** | **NPs@silica** | 1 | 23.8 | 63.8 | 12.5 | 0 | 0 | 37.5 | 3.4 | 1024 |
| **20-04** | **NPs@silica** | 1 | 48 | 32 | 0 | 20 | 0 | 46.0 | 16.6 | 1606 |
| **23-02** | **NPs@silica** | 1 | 36 | 55 | 0 | 9 | 0 | 54.0 | 7.7 | 1000 |
| **23-08** | **NPs@tenside** | 2 | 36 | 55 | 0 | 9 | 0 | 33.0 | 10,3 | 615 |
| **47-01** | **NPs@silica** | 1 | 52 | 29 | 0 | 0 | 19 | 39.0 | 7.3 | 405 |

### Characterization methods

For particle size analysis a Mastersizer 2000 with Hydro 2000 (Malvern Instruments) was used. Samples were suspended in water or ethanol prior to measurements.

For surface area and pore size analysis an Autosorb iQ (Quantachrome Instruments) was used. Samples were degased at 95 °C for 12 h under vacuum for activation.

For magnetization measurements a 7404-S Magnetometer (Lake Shores Cryotronics) was used.

One key requirement for the automation of the particles inside the enrichment-workflow-MS technology is a fast magnetic separation (<5 s) for high throughput. Particle size and saturation magnetization are crucial properties. Therefore particles with high saturation magnetization (>1 A m² kg⁻¹) and large sizes (>2 µm) are required. Additionally, for the robustness of the system, carry-over of particles has to be avoided. Therefore the particles need to have high magnetization and particle sizes larger than 1µm.

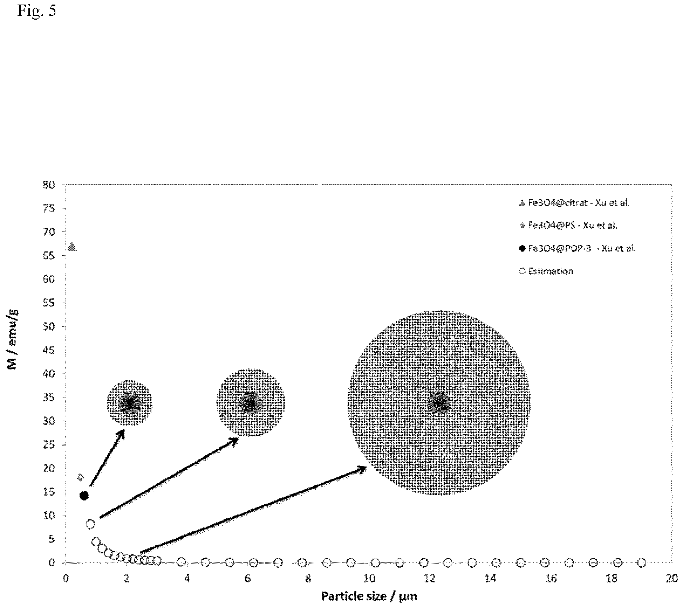

This is the main problem of the just described synthesis routes of magnetic polymer particles. The method of Yang *et al.* shows the synthesis of small particles (< 100 nm) with a low saturation magnetization (4,1 A m² kg⁻¹). These particles do not meet the requirements for automation due to small particle size and low magnetization properties. It is difficult to produce larger particles than 1 µm by emulsion polymerization, giving a low potential of this synthesis method for the production of particles with the required properties. Xu *et al.* shows the synthesis of small particles (about 400 nm) with a high saturation magnetization (14,1 A m² kg⁻¹). With this method, due to the fact that only one Fe₃O₄-NP core is embedded per polymer particle (mono-core), larger particles can only be produced by increasing the polymer shell, which would lead to a drastically decrease of the saturation magnetization. Figure 5 shows the calculated saturation magnetization for the particles synthesized by Xu *et al.* with different sizes. For example for a synthesis of particles with a size of 2 µm the saturation magnetization would be below 1 A m² kg⁻¹ and for 10 µm lower than 0.01 A m² kg⁻¹. This significant loss of magnetization would lead to extremely long separation times, which are not accepted for high throughput automation. Therefore, these particles do not meet the requirements for automation due to small particle size and mono-core submicron particles synthesis which leads to low magnetization properties.

After polymerization, there is a hypercrosslinking reaction necessary to produce porosity in the particle and therefore high surface areas. One drawback is the building of HCl during this chemical reaction. HCl attacks the Fe₃O₄-NPs and therefore lowers the magnetization of the particles. For this reaction step, the coating thickness and the type of coating of the Fe₃O₄-NPs are essential. Yang *et al.* uses coating with oleic acid and Xu *et al.* protects the Fe₃O₄-NPs with citrate.

### Example 2: Analyte capturing using magnetic beads

### Analyte capturing

For the bead evaluation the beads underwent the enrichment workflow as illustrated in Fig-ure 6. As test samples a spiked serum pool was used, where the spiked analytes are listed in the following Table 2.

**Table 2: List of analytes tested with CPS particles**

| **Steroids** | **TDMs** | **DATs** | **Vitamines** |
|---|---|---|---|
| Testosterone | Gabapentin Valproic acid Cyclosporin Everolimus Gentamicin Tobramycin | Methampheta-mine THC Chlordiazepoxid Midazolam Nordiazepam | 25OH-VitD₃ |

### Preparation of samples

Samples were prepared by spiking the 13 analytes of interest into an analyte-free human serum pool. Internal standard solution was a methanol/water 50:50 v/v mixture containing isotope labelled analogues of the target analytes.

### Bead extraction

For each sample, 100 µL of serum were mixed with 50 µL of bead suspension in water at a concentration of 50 mg/mL and equilibrated for 5 min at room temperature under gentle rolling conditions, so that the analytes could access the entire surface of the particles. In cases where 25OH-Vitamin D₃ was analyzed, an additional step was included where 100 µL pre-treatment solution was added to the mixture and incubated for 15 min. The supernatant was then discarded and the magnetic beads washed twice with 200 µL of water. Elution took place with 100 µL acetonitrile/2% formic acid in water 70:30 v/v. In the next step, 80 µL eluate were withdrawn from the vial and transferred to an HPLC vial, where 5 µL internal standard solution were added prior to LC-MS/MS analysis.

### Recovery

Quantification was performed by external calibration. For this, a calibration curve was recorded in neat solution. Recovery was calculated by comparing the calculated concentration in the eluate fraction to the spiked amount.

A further aspect to be characterized was the eluate purity regarding residual protein as classified according to:

| | | | |
|---|---|---|---|
| Residual protein: | Good | < 1.0% | (< 855 µg/mL) |
| | Medium | 1.0 - 2.0% | (855 - 1710 µg/mL) |
| | Poor | > 2.0% | (> 1710 µg/mL) |

For almost all samples the residual protein was below 1% showing good results for the re-movement of matrix effects.

The following Table 3 shows the capture properties of the different bead types presented above. As can be seen, that with different monomer constitutions, the capture efficiency for different analyte classes changes, giving the possibility of tailored bead design.

**Table 3: Capture properties of different beads**

| **Bead Type** | **Genta micin** | **Gabapentin** | **Methamphetamine** | **Chlordiazepoxid** | **Valproic acid** | **Midazolam** | **Tobra mycin** | **Nordiazepam** | **Cyclosporin** | **THC** | **Everoli mus** | **Testosterone** | **VitD** |
|---|---|---|---|---|---|---|---|---|---|---|---|---|---|
| | % | % | % | % | % | % | % | % | % | % | % | % | % |
| **01-03** | 76% | 10% | 92% | 83% | 14% | 81% | 59% | 76% | 58% | 26% | 54% | 65% | - |
| **01-05** | 78% | 17% | 76% | 71% | 9% | 71% | 59% | 60% | 40% | 13% | 40% | 50% | 4% |
| **01-10** | 87% | 40% | 111% | 100% | 26% | 80% | 38% | 71% | 35% | 8% | 30% | 57% | 0% |
| **04-13** | 91% | 33% | 91% | 81% | 28% | 77% | 75% | 67% | 31% | 10% | 36% | 48% | - |
| **04-12** | 82% | 26% | 90% | 81% | 28% | 77% | 59% | 64% | 35% | 8% | 38% | 49% | 0% |
| **33-02** | 54% | 15% | 81% | 92% | 12% | 80% | 17% | 77% | 50% | 11% | 12% | 68% | - |
| **33-05** | 16% | 4% | 93% | 118% | 4% | 96% | 31% | 99% | 59% | 11% | 25% | 64% | 0% |
| **44-01** | 42% | 11% | 81% | 76% | 13% | 72% | 14% | 67% | 38% | 6% | 5% | 57% | 17%* |
| **50-01** | 34% | 15% | 75% | 70% | 17% | 77% | 19% | 70% | 34% | 8% | 18% | 51% | 2% |
| **05-22** | 50% | 11% | 87% | 41% | 3% | 19% | 46% | 48% | 68% | 12% | 51% | 57% | - |
| **05-26** | 53% | 2% | 87% | 65% | 3% | 30% | 19% | 62% | 74% | 14% | 26% | 60% | 2% |
| **05-27** | 70% | 2% | 94% | 70% | 2% | 35% | 32% | 56% | 38% | 12% | 20% | 52% | 0% |
| **05-36** | 54% | 4% | 81% | 89% | 4% | 37% | 24% | 72% | 30% | 5% | 24% | 9% | 0% |
| **39-01** | 18% | 3% | 82% | 87% | 2% | 79% | 15% | 79% | 58% | 24% | 14% | 68% | 10%* |
| **40-01** | 33% | 13% | 80% | 85% | 13% | 79% | 8% | 79% | 34% | 6% | 7% | 67% | 4%* |
| **42-01** | 46% | 19% | 79% | 82% | 16% | 76% | 13% | 73% | 28% | 4% | 4% | 55% | 19%* |
| **43-01** | 31% | 19% | 64% | 74% | 16% | 66% | 11% | 66% | 22% | 7% | 4% | 51% | 11%* |
| **46-01** | 3% | 1% | 67% | 82% | 2% | 51% | 5% | 72% | 57% | 7% | 16% | 30% | 34%* |
| **48-01** | 28% | 10% | 79% | 73% | 12% | 77% | 19% | 72% | 39% | 9% | 25% | 51% | 4% |
| **49-01** | 38% | 15% | 58% | 53% | 13% | 57% | 12% | 53% | 26% | 7% | 16% | 39% | 0% |
| **51-01** | 44% | 16% | 63% | 50% | 17% | 59% | 20% | 53% | 29% | 5% | 15% | 43% | 2% |
| **20-04** | 60% | 33% | 61% | 47% | 19% | 41% | 57% | 33% | 6% | 3% | 4% | 18% | - |
| **23-02** | 81% | 21% | 94% | 86% | 36% | 70% | 64% | 73% | 36% | 5% | 44% | 49% | - |
| **47-01** | 15% | 4% | 93% | 142% | 5% | 103% | 32% | 107% | 54% | 6% | 32% | 48% | 0% |

## Claims

1. Magnetic particle comprising a polymer matrix (P) and at least one magnetic core (M), preferably at least two magnetic cores,
wherein the polymer matrix comprises a hypercrosslinked polymer,
wherein the magnetic particle has a particle size in the range of from 5 to 40 micrometers, as determined according to ISO 13320;
wherein the polymer matrix (P) comprises a co-polymer obtained or obtainable by a method comprising co-polymerizing suitable monomeric building blocks in the presence of one monomeric building block which is a crosslinking agent, wherein 5-90 vol% of all monomeric building blocks are divinylbenzenes;
wherein in the co-polymerization a polymer with a crosslinking degree of at least 5% is obtained;
wherein divinylbenzene is the crosslinking agent
wherein the suitable monomeric building blocks are selected from the group consisting of styrene, functionalized styrenes, vinylbenzylchloride, divinylbenzene, vinylacetate, methylmethaacrylate and acrylic acid;
wherein the at least one magnetic core (M) comprises at least one iron oxide nano-particle;
wherein the at least one magnetic core (M) comprises at least one magnetic nano-particle and a coating C1;
wherein the coating C1 is tenside or silica coating.

2. Magnetic particle of claim 1, wherein the polymer matrix comprises pores having a pore size smaller than 100 nm, preferably smaller or equal to 50 nm, as determined according to ISO 15901-3.

3. Magnetic particle of claim 2, wherein at least 90% of all pores present in the polymer matrix (P) have a pore size smaller than 10 nm and at least 50% of all pores present in the polymer matrix have a pore size smaller than 5 nm, as determined according to ISO 15901-3, preferably, wherein the polymer matrix (P) does not comprise macropores having a pore size larger than 50 nm.

4. Magnetic particle of any one of claims 1 to 3, wherein the magnetic particle has a saturation magnetization of at least 1 A m²/kg, preferably, of at least 10 A m²/kg.

5. Magnetic particle of any one of claims 1 to 4, wherein the magnetic particle is superparamagnetic.

6. Magnetic particle of any one of claims 1 to 5, wherein the at least one magnetic core (M) comprises a Fe₃O₄-nanoparticle, .

7. Magnetic particle of any one of claims 1 to 5, wherein the at least one magnetic core (M) comprises, preferably consists of, a supraparticle.

8. Magnetic particle of claim 7, wherein said supraparticle consists of aggregated nanoparticles and, preferably, of more than 20 aggregated nanoparticles and, more preferably, of between 100 and 1.5 million nanoparticles.

9. Magnetic particle of claim 7 or 8, wherein the at least one magnetic core (M) comprises, preferably consists of, a supraparticle and at least one coating C2, which coating preferably is deposited on the surface of the nanoparticles,
and wherein the coating C2 is preferably selected from the group consisting of dicarboxylic acids, dicarboxylic acid salts, dicarboxylic acid derivatives, polyacrylic acid, polyacrylic acid salts, polyacrylic acid derivatives, tricarboxylic acids, tricarboxylic acid salts, tricarboxylic derivatives, amino acids, amino acid salts, amino acid derivatives, surfactants, salt of surfactants, fatty acids, fatty acid salts and fatty acid derivatives.

10. A method of preparing a magnetic particle comprising a polymer matrix (P) and at least one magnetic core (M), preferably at least two magnetic cores (M), wherein the polymer matrix (P) comprises at least one crosslinked polymer, wherein the magnetic particle has a particle size in the range of from 5 to 40 micrometers, as determined according to ISO 13320, the method comprising:
(i) providing at least one magnetic core (M), preferably at least two magnetic cores (M),
(ii) providing polymer precursor molecules,
(iii) polymerizing the polymer precursor molecules according to (ii) in the presence of the at least one magnetic core (M), thereby forming a particle comprising the at least one magnetic core (M), preferably the at least two magnetic cores (M), embedded in a polymer matrix (P1), wherein the polymer matrix (P1) comprises, more preferably consists of a crosslinked polymer, and
(iv) hypercrosslinking the polymer matrix (P1) of the polymer particle obtained in (iii),
to give the magnetic particle;
wherein the reaction in (iv) is not carried out in a solvent comprising dichloroethane or other organic halides;
wherein in (iii) the polymer matrix (P1) comprises a crosslinked polymer being obtained or obtainable by crosslinking a polymer with 5-90 vol% of a divinylbenzene crosslinking agent, based on the total amount of the polymer, wherein in the resulting polymer a crosslinking degree of at least 5% is obtained;
wherein divinylbenzene is the crosslinking agent;
wherein the hypercrosslinking, in (iv) is carried out at a temperature in the range of from -30 to 120°C;
wherein the reaction in (iv) is carried out in a solvent comprising at least THF, acetonitrile, DMF, dioxane or toluol.

11. Magnetic particle obtained or obtainable by a method of claim 10.

12. Use of the magnetic particle of any one of claims 1 to 9 or a magnetic particle of claim 11 for qualitative and/or quantitative determination of at least one analyte in a fluid.

13. A method for determining at least one analyte in a fluid sample comprising the steps of:
a) contacting a magnetic particle of any one of claims 1 to 9 or claim 11 with a fluid sample comprising or suspected to comprise the at least one analyte; and
b) determining the at least one analyte bound to the said magnetic particle.

## Patentansprüche

1. Magnetpartikel, umfassend eine Polymermatrix (P) und mindestens einen Magnetkern (M), vorzugsweise mindestens zwei Magnetkerne,
wobei die Polymermatrix ein hypervernetztes Polymer umfasst,
wobei das Magnetpartikel eine Partikelgröße im Bereich von 5 bis 40 Mikrometer aufweist, wie gemäß ISO 13320 bestimmt;
wobei die Polymermatrix (P) ein Copolymer umfasst, das durch ein Verfahren erhalten wird oder erhältlich ist, umfassend das Copolymerisieren geeigneter monomerer Bausteine in Gegenwart eines monomeren Bausteins, der ein Vernetzungsmittel ist, wobei 5-90 Vol.-% aller monomeren Bausteine Divinylbenzole sind;
wobei bei der Copolymerisation ein Polymer mit einem Vernetzungsgrad von mindestens 5 % erhalten wird;
wobei Divinylbenzol das Vernetzungsmittel ist,
wobei die geeigneten monomeren Bausteine ausgewählt sind aus der Gruppe, bestehend aus Styrol, funktionalisierten Styrolen, Vinylbenzylchlorid, Divinylbenzol, Vinylacetat, Methylmethacrylat und Acrylsäure;
wobei der mindestens eine Magnetkern (M) mindestens ein Eisenoxidnanopartikel umfasst;
wobei der mindestens eine Magnetkern (M) mindestens ein magnetisches Nanopartikel und eine Beschichtung C1 umfasst;
wobei die Beschichtung C1 eine Tensid- oder Siliciumdioxidbeschichtung ist.

2. Magnetpartikel nach Anspruch 1, wobei die Polymermatrix Poren mit einer Porengröße von kleiner als 100 nm, vorzugsweise kleiner oder gleich 50 nm, umfasst, bestimmt gemäß ISO 15901-3.

3. Magnetpartikel nach Anspruch 2, wobei mindestens 90 % aller in der Polymermatrix (P) vorhandenen Poren eine Porengröße von kleiner als 10 nm aufweisen und mindestens 50 % aller in der Polymermatrix vorhandenen Poren eine Porengröße von kleiner als 5 nm aufweisen, bestimmt gemäß ISO 15901-3, vorzugsweise wobei die Polymermatrix (P) keine Makroporen mit einer Porengröße von größer als 50 nm umfasst.

4. Magnetpartikel nach einem der Ansprüche 1 bis 3, wobei das Magnetpartikel eine Sättigungsmagnetisierung von mindestens 1 A m²/kg, vorzugsweise von mindestens 10 A m²/kg aufweist.

5. Magnetpartikel nach einem der Ansprüche 1 bis 4, wobei das Magnetpartikel superparamagnetisch ist.

6. Magnetpartikel nach einem der Ansprüche 1 bis 5, wobei der mindestens eine Magnetkern (M) ein Fe₃O₄-Nanopartikel umfasst.

7. Magnetpartikel nach einem der Ansprüche 1 bis 5, wobei der mindestens eine Magnetkern (M) ein Suprapartikel umfasst, vorzugsweise daraus besteht.

8. Magnetpartikel nach Anspruch 7, wobei das Suprapartikel aus aggregierten Nanopartikeln und vorzugsweise aus mehr als 20 aggregierten Nanopartikeln und noch bevorzugter aus zwischen 100 und 1,5 Millionen Nanopartikeln besteht.

9. Magnetpartikel nach Anspruch 7 oder 8, wobei der mindestens eine Magnetkern (M) ein Suprapartikel und mindestens eine Beschichtung C2 umfasst, vorzugsweise daraus besteht, wobei die Beschichtung vorzugsweise auf der Oberfläche der Nanopartikel abgeschieden ist,
und wobei die Beschichtung C2 vorzugsweise ausgewählt ist aus der Gruppe, bestehend aus Dicarbonsäuren, Dicarbonsäuresalzen, Dicarbonsäurederivaten, Polyacrylsäure, Polyacrylsäuresalzen, Polyacrylsäurederivaten, Tricarbonsäuren, Tricarbonsäuresalzen, Tricarbonsäurederivaten, Aminosäuren, Aminosäuresalzen, Aminosäurederivaten, Tensiden, Salz von Tensiden, Fettsäuren, Fettsäuresalzen und Fettsäurederivaten.

10. Verfahren zur Herstellung eines Magnetpartikels, umfassend eine Polymermatrix (P) und mindestens einen Magnetkern (M), vorzugsweise mindestens zwei Magnetkerne (M), wobei die Polymermatrix (P) mindestens ein vernetztes Polymer umfasst, wobei das Magnetpartikel eine Partikelgröße im Bereich von 5 bis 40 Mikrometer aufweist, bestimmt gemäß ISO 13320 , wobei das Verfahren Folgendes umfasst:
(i) Bereitstellen mindestens eines Magnetkerns (M), vorzugsweise mindestens zweier Magnetkerne (M),
(ii) Bereitstellen von Polymervorläufermolekülen,
(iii) Polymerisieren der Polymervorläufermoleküle gemäß (ii) in Gegenwart des mindestens einen Magnetkerns (M), wodurch ein Partikel gebildet wird, umfassend den mindestens einen Magnetkern (M), vorzugsweise die mindestens zwei Magnetkerne (M), eingebettet in eine Polymermatrix (P1), wobei die Polymermatrix (P1) ein vernetztes Polymer umfasst, noch bevorzugter daraus besteht, und
(iv) Hypervernetzen der Polymermatrix (P1) des in (iii) erhaltenen Polymerpartikels, unter Bildung des Magnetpartikels;
wobei die Reaktion in (iv) nicht in einem Lösungsmittel durchgeführt wird, das Dichlorethan oder andere organische Halogenide umfasst;
wobei in (iii) die Polymermatrix (P1) ein vernetztes Polymer umfasst, das durch Vernetzen eines Polymers mit 5-90 Vol.-% eines Divinylbenzol-Vernetzungsmittels, bezogen auf die Gesamtmenge des Polymers, erhalten wird oder erhältlich ist, wobei in dem resultierenden Polymer ein Vernetzungsgrad von mindestens 5 % erhalten wird;
wobei Divinylbenzol das Vernetzungsmittel ist;
wobei das Hypervernetzen in (iv) bei einer Temperatur im Bereich von -30 bis 120 °C durchgeführt wird;
wobei die Reaktion in (iv) in einem Lösungsmittel durchgeführt wird, das mindestens THF, Acetonitril, DMF, Dioxan oder Toluol umfasst.

11. Magnetpartikel, erhalten oder erhältlich durch ein Verfahren nach Anspruch 10.

12. Verwendung des Magnetpartikels nach einem der Ansprüche 1 bis 9 oder eines Magnetpartikels nach Anspruch 11 zur qualitativen und/oder quantitativen Bestimmung mindestens eines Analyten in einem Fluid.

13. Verfahren zur Bestimmung mindestens eines Analyten in einer Fluidprobe, umfassend die folgenden Schritte:
a) Inkontaktbringen eines Magnetpartikels nach einem der Ansprüche 1 bis 9 oder Anspruch 11 mit einer Fluidprobe, die den mindestens einen Analyten umfasst oder von der vermutet wird, dass sie den mindestens einen Analyten umfasst; und
b) Bestimmen des mindestens einen Analyten, der an das Magnetpartikel gebunden ist.

## Revendications

1. Particule magnétique comprenant une matrice polymère (P) et au moins un noyau magnétique (M), de préférence au moins deux noyaux magnétiques,
dans laquelle la matrice polymère comprend un polymère hyperréticulé,
dans laquelle la particule magnétique a une taille de particule dans la plage allant de 5 à 40 micromètres, comme déterminé selon la norme ISO 13320 ;
dans laquelle la matrice polymère (P) comprend un copolymère obtenu ou pouvant être obtenu par un procédé comprenant la copolymérisation de blocs de construction monomères appropriés en présence d'un bloc de construction monomère qui est un agent de réticulation, dans laquelle 5 à 90 % en volume de tous les blocs de construction monomères sont des divinylbenzènes ;
dans laquelle lors de la copolymérisation un polymère avec un degré de réticulation d'au moins 5 % est obtenu ;
dans laquelle le divinylbenzène est l'agent de réticulation ;
dans laquelle les blocs de construction monomères appropriés sont choisis dans le groupe constitué par le styrène, les styrènes fonctionnalisés, le chlorure de vinylbenzyle, le divinylbenzène, l'acétate de vinyle, le méthacrylate de méthyle et l'acide acrylique ;
dans laquelle l'au moins un noyau magnétique (M) comprend au moins une nanoparticule d'oxyde de fer ;
dans laquelle l'au moins un noyau magnétique (M) comprend au moins une nanoparticule magnétique et un revêtement C1 ;
dans laquelle le revêtement C1 est un revêtement de tenside ou de silice.

2. Particule magnétique selon la revendication 1, dans laquelle la matrice polymère comprend des pores ayant une taille de pore inférieure à 100 nm, de préférence inférieure ou égale à 50 nm, comme déterminé selon la norme ISO 15901-3.

3. Particule magnétique selon la revendication 2, dans laquelle au moins 90 % de tous les pores présents dans la matrice polymère (P) ont une taille de pore inférieure à 10 nm et au moins 50 % de tous les pores présents dans la matrice polymère ont une taille de pore inférieure à 5 nm, comme déterminé selon la norme ISO 15901-3, de préférence, dans laquelle la matrice polymère (P) ne comprend pas de macropores ayant une taille de pore supérieure à 50 nm.

4. Particule magnétique selon l'une quelconque des revendications 1 à 3, dans laquelle la particule magnétique a une magnétisation de saturation d'au moins 1 A m²/kg, de préférence, d'au moins 10 A m²/kg.

5. Particule magnétique selon l'une quelconque des revendications 1 à 4, dans laquelle la particule magnétique est superparamagnétique.

6. Particule magnétique selon l'une quelconque des revendications 1 à 5, dans laquelle l'au moins un noyau magnétique (M) comprend une nanoparticule de Fe₃O₄.

7. Particule magnétique selon l'une quelconque des revendications 1 à 5, dans laquelle l'au moins un noyau magnétique (M) comprend, de préférence est constitué de, une supraparticule.

8. Particule magnétique selon la revendication 7, dans laquelle ladite supraparticule est constituée de nanoparticules agrégées et, de préférence, de plus de 20 nanoparticules agrégées et, plus préférentiellement, d'entre 100 et 1,5 million de nanoparticules.

9. Particule magnétique selon la revendication 7 ou 8, dans laquelle l'au moins un noyau magnétique (M) comprend, de préférence est constitué de, une supraparticule et au moins un revêtement C2, lequel revêtement est de préférence déposé sur la surface des nanoparticules,
et dans laquelle le revêtement C2 est de préférence choisi dans le groupe constitué par les acides dicarboxyliques, les sels d'acides dicarboxyliques, les dérivés d'acides dicarboxyliques, l'acide polyacrylique, les sels d'acide polyacrylique, les dérivés d'acide polyacrylique, les acides tricarboxyliques, les sels d'acides tricarboxyliques, les dérivés tricarboxyliques, les acides aminés, les sels d'acides aminés, les dérivés d'acides aminés, les tensioactifs, les sels de tensioactifs, les acides gras, les sels d'acides gras et les dérivés d'acides gras.

10. Procédé de préparation d'une particule magnétique comprenant une matrice polymère (P) et au moins un noyau magnétique (M), de préférence au moins deux noyaux magnétiques (M), dans lequel la matrice polymère (P) comprend au moins un polymère réticulé, dans lequel la particule magnétique a une taille de particule dans la plage allant de 5 à 40 micromètres, comme déterminé selon la norme ISO 13320, le procédé comprenant :
(i) la fourniture d'au moins un noyau magnétique (M), de préférence d'au moins deux noyaux magnétiques (M),
(ii) la fourniture de molécules précurseurs de polymères,
(iii) la polymérisation des molécules précurseurs de polymères selon (ii) en présence de l'au moins un noyau magnétique (M), formant ainsi une particule comprenant l'au moins un noyau magnétique (M), de préférence les au moins deux noyaux magnétiques (M), intégrés dans une matrice polymère (P1), la matrice polymère (P1) comprenant, plus préférentiellement étant constituée d'un polymère réticulé, et
(iv) l'hyperréticulation de la matrice polymère (P1) de la particule polymère obtenue dans (iii),
pour donner la particule magnétique ;
dans lequel la réaction de (iv) n'est pas réalisée dans un solvant comprenant du dichloroéthane ou d'autres halogénures organiques ;
dans lequel dans (iii) la matrice polymère (P1) comprend un polymère réticulé obtenu ou pouvant être obtenu par réticulation d'un polymère avec 5 à 90 % en volume d'un agent de réticulation divinylbenzène, en se basant sur la quantité totale du polymère, dans lequel dans le polymère résultant un degré de réticulation d'au moins 5 % est obtenu ;
dans lequel le divinylbenzène est l'agent de réticulation ;
dans lequel l'hyperréticulation, dans (iv), est réalisée à une température dans la plage allant de -30 à 120 °C ;
dans lequel la réaction dans (iv) est réalisée dans un solvant comprenant au moins le THF, l'acétonitrile, le DMF, le dioxane ou le toluène.

11. Particule magnétique obtenue ou pouvant être obtenue par un procédé selon la revendication 10.

12. Utilisation de la particule magnétique selon l'une quelconque des revendications 1 à 9 ou d'une particule magnétique selon la revendication 11 pour la détermination qualitative et/ou quantitative d'au moins un analyte dans un fluide.

13. Procédé de détermination d'au moins un analyte dans un échantillon de fluide comprenant les étapes de :
a) mise en contact d'une particule magnétique selon l'une quelconque des revendications 1 à 9 ou la revendication 11 avec un échantillon de fluide comprenant ou suspecté de comprendre l'au moins un analyte ; et
b) détermination de l'au moins un analyte lié à ladite particule magnétique.
